# EUROPEAN PATENT APPLICATION

(11) **EP 4 721 759 A1**
(43) Date of publication of application: **08.04.2026**
(21) Application number: 24799890.9
(22) Date of filing: 29.04.2024
(51) Int. Cl.: A61K 47/18, A61K 47/28, A61K 9/51, A61K 48/00, A61P 35/00, A61P 39/00, A61P 31/04, A61P 31/10

(54) **LIPID NANOPARTICLE FOR DELIVERING NUCLEIC ACID DRUG, AND PREPARATION METHOD THEREFOR AND USE THEREOF**

(30) Priority: 29.04.2023 CN 202310482077
(71) Applicant: Cansino (Shanghai) Biological Research Co., Ltd., Shanghai 201203 (CN)
(72) Inventor: WANG, Zhenghua, Shanghai 201203 (CN); WANG, Haomeng, Shanghai 201203 (CN); YAN, Zhihong, Shanghai 201203 (CN); DAI, Shouyi, Shanghai 201203 (CN); LIU, Jian, Shanghai 201203 (CN); QIU, Dongxu, Shanghai 201203 (CN); ZHU, Tao, Shanghai 201203 (CN); YU, Xuefeng, Shanghai 201203 (CN)
(74) Representative: Finnegan Europe LLP
(86) International application number: PCT/CN2024/090657
(87) International publication number: WO 2024/227433

(57) **Abstract**

A lipid nanoparticle (LNP) composition for delivering a nucleic acid drug, and a preparation method therefor and a use thereof. The composition prepared by mixing a steroid-cationic lipid compound with a helper lipid and a polyethylene glycol modified phospholipid has better stability and transfection efficiency. The use of an LNP to deliver a nucleic acid, such as mRNA, can efficiently and stably deliver a nucleic acid drug to a target cell or organ, and meanwhile, the LNP can be used for aerosol inhalation administration of the mRNA and can also be used for developing a lyophilized preparation of the mRNA. A higher specific antibody response can be induced in an experimental animal, and the compound has better safety.

## Description

### TECHNICAL FIELD

The present disclosure pertains to the field of biopharmaceuticals, and particularly relates to a lipid nanoparticle for nucleic acid drug delivery, a preparation method therefor, and use thereof.

### BACKGROUND

Nucleic acid drugs primarily refer to compounds containing nucleotide or deoxynucleotide structures with genetic characteristics and pharmacological activity, which can be used for treating tumors, tissue regeneration, wound healing, pulmonary fibrosis, inflammatory diseases, microbial infections, etc. After being injected into the human body, the nucleic acid drugs require an efficient and safe drug delivery system to deliver them to lesion sites. This drug delivery system needs to remain in the body for a sufficient duration to accurately target the lesion sites while avoiding damage to normal cells. Currently, drug delivery systems may be categorized into viral carriers and non-viral carriers. Viral carriers have relatively fewer applications in nucleic acid drugs due to their immunogenicity, tumorigenicity, and limited drug loading; non-viral carriers, such as polymers and lipids (liposomes or LNPs), can bind nucleic acid drugs to specific ligands to enable them to target specific cells, and have relatively more applications in current nucleic acid drugs. At present, LNPs are one of the most commonly used delivery systems for nucleic acid drug research, and LNP delivery systems can safely and effectively deliver nucleic acids. They have the advantages of high nucleic acid encapsulation efficiency, capability of effective cell transfection, strong tissue penetration, low cytotoxicity and immunogenicity, and the like, which facilitate drug delivery. Compared with other drug delivery systems, LNP delivery systems are highly advantageous. Therefore, LNP delivery systems have broad development and application prospects.

In the prior art, LNP delivery systems are often made from ingredients such as ionizable lipids (cationic lipids), steroids, neutral lipids, polyethylene glycol lipids, nucleic acid drugs, and the like. For example, the patent document AU2020325221A1 discloses a composition of target cell delivery LNPs, which comprises: (i) an ionizable lipid; (ii) a sterol or other structural lipid; (iii) a non-cationic helper lipid or phospholipid; (iv) a PEG lipid; and (v) an agent (e.g., a nucleic acid molecule) encapsulated in and/or associated with the LNP, where the four components are assembled in a specific ratio to enhance the efficiency of delivery to target cells. The patent document WO2021/250263 A1 discloses a composition comprising an ionizable lipid, a phospholipid, a sterol, a polyethylene glycol lipid, and one or more nucleic acids, and discloses a composition comprising less than about 1 mol% of a C14-PEG2000 lipid and a specific percentage of other lipids. The patent CN102712935B discloses a lipid particle comprising: a cationic lipid; a neutral lipid, a zwitterionic lipid, or an anionic lipid; a polyethylene glycol lipid; a sterol; and a nucleic acid, where the above components are assembled into a lipid particle having a solid core, and the solid core can achieve higher coating efficiency. The patent document WO 2021/055849A1 discloses a lipid having the following structure: 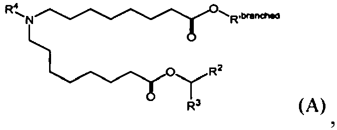 and the structure can improve its safety, effectiveness, and specificity. The patent document WO2021/026358A1 discloses a target cell delivery lipid nanoparticle (LNP), comprising: (i) an ionizable lipid; (ii) a sterol or other structural lipid; (iii) a non-cationic helper lipid or phospholipid; (iv) a payload; and (v) a polyethylene glycol lipid, which is used as a drug delivery system to allow for safety and effectiveness. In recent years, it has been found that ionizable lipid compounds with the introduction of cholesterol can also be used in nucleic acid drug delivery. The patent document US7514099B2 discloses a cholesterol amino lipid compound CLinDMA 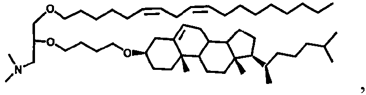 which can form a four-component LNP with a phospholipid, cholesterol, and a polyethylene glycol lipid, or form a five-component LNP with a phospholipid, a DMOBA lipid, cholesterol, and a PEG lipid to deliver an siRNA. The patent CN112424214A discloses an ionizable cationic lipid compound (3) 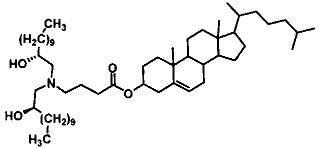 formed from cholesterol and a linear olefin, which forms a lipid nanoparticle with cholesterol, DPPC, DOPE, and DMG-PEG200 to deliver a nucleic acid; the construction needs 5 lipid excipients and is complex, and the compound has a relatively poor polydispersity index (PDI) of -0.292. In order to adapt to different application scenarios, it is also particularly important to develop different delivery systems suitable for different routes of administration, such as the use of aerosol inhalation administration in treating lung-related diseases or preventing infections caused by respiratory tract-related pathogens. At present, the stable storage of mRNA-LNPs remains a major difficulty, and mRNA-LNPs can be stably stored at 2-8 °C or even at room temperature by lyophilization. It can be concluded that, in order to further obtain LNP delivery systems that are safer, more efficient, and more stable, have simple construction, and can be suitable for use in different routes of administration simultaneously, their components and the structures of the components need to be further developed, by optimizing the components and optimizing the cationic lipids in the composition in the prior art.

Conventional LNPs comprise 4 components: ionizable cationic lipids, amphiphilic phospholipids, PEGylated lipids, and cholesterol. mRNAs, encapsulated in the conventional LNPs, must be stored and transported in an ultra-low temperature environment due to their characteristics. According to current studies, the COVID-19 mRNA vaccine from Pfizer can be stored for 6 months in an ultra-low temperature freezer at -70 °C, for 15 days in a customized thermostatic transport box from Pfizer with continuous dry ice addition, and for only 5 days in an ordinary hospital refrigerator at 2-8 °C. Storage at such an extremely low temperature as -70 °C is very common in research laboratories, but such devices are unavailable in many medical centers, and even scarcer in developing countries and economically less developed regions. At present, there is no existing capacity in this field in China for transportation that can maintain a -70 °C environment. Therefore, it is necessary to address the storage problem of nucleic acid vaccines.

### SUMMARY

The present disclosure provides a lipid nanoparticle composition. Differing from conventional 4-component LNPs, the present disclosure covalently binds an ionizable cationic lipid to a steroid and then assembles them with a helper lipid and a PEGylated lipid into a lipid nanoparticle, thereby forming a 3-component lipid nanoparticle composition, which has good lyophilization stability and can meet the requirement of storage at 2-8 °C.

In a first aspect, the present disclosure provides a lipid nanoparticle composition for nucleic acid drug delivery, comprising: a steroid-cationic lipid compound, a helper lipid, and a polyethylene glycol lipid.

In one embodiment, the molar ratio of the steroid-cationic lipid to the helper lipid to the polyethylene glycol lipid is (30-80):(20-80):(0.5-20); preferably, the molar ratio of the steroid-cationic lipid to the helper lipid to the polyethylene glycol lipid is (30-80):(30-80):(0.5-20).

In one embodiment, the polyethylene glycol lipid is selected from 2-[(polyethylene glycol)-2000]-N,N-ditetradecylacetamide (ALC-0159), 1,2-dimyristoyl-sn-glycero-methoxypolyethylene glycol (PEG-DMG), 1,2-distearoyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearoyl, PEG-diacylglycerol amide (PEG-DAG), PEG-dipalmitoyl phosphatidylethanolamine (PEG-DPPE), and PEG-1,2-dimyristoyloxypropyl-3-amine (PEG-c-DMA).

In one embodiment, the helper lipid is selected from a neutral lipid, a zwitterionic lipid, and an anionic lipid; preferably, the helper lipid is selected from 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoylphosphatidylcholine (POPC), 1-palmitoyl-2-oleoylphosphatidylethanolamine (POPE), phosphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauroylphosphatidylcholine (DLPC), dimyristoylphosphatidylcholine (DMPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1,2-diarachidoyl-sn-glycero-3-phosphocholine (DBPC), 1-stearoyl-2-palmitoylphosphatidylcholine (SPPC), 1,2-eicosenoyl-sn-glycero-3-phosphocholine (DEPC), lysophosphatidylcholine, dilinoleoylphosphatidylcholine distearoylphosphatidylethanolamine (DSPE), and lysophosphatidylethanolamine.

In one embodiment, the composition further comprises a nucleic acid drug; preferably, the nucleic acid drug is a DNA or an RNA; more preferably, the RNA is selected from an antisense RNA, an saRNA, an mRNA, an lncRNA, an miRNA, an siRNA, a piRNA, a gRNA, a tsRNA, a circRNA, and a self-amplifying mRNA; more preferably, the nucleic acid drug is an mRNA; more preferably, the composition is in the form of a nucleic acid-lipid particle.

In one embodiment, the steroid-cationic lipid compound in the composition has a structure represented by formula (I): 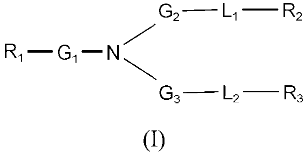
wherein R₁ is selected from -OR₄, -NR₄R₅, -NR₄C(=O)R₅, -C(=O)OR₅, -OC(=O)R₅, -OC(=O)OR₅, - CN, a nitrogen-containing heterocyclic group, and guanidino;
R₄ and R₅ are each independently selected from H, C₁₋₉ alkyl, C₂₋₉ alkenyl, C₂₋₉ alkynyl, C₃₋₈ cycloalkyl, C₃₋₈ cycloalkenyl, and C₃₋₈ cycloalkynyl;
G₁ is selected from a chemical bond (-), C₁₋₉ alkylene, C₂₋₉ alkenylene, C₃₋₉ alkynylene, C₃₋₈ cycloalkylene, and C₃₋₈ cycloalkenylene;
preferably, R₁-G₁- has no more than 10 contiguous carbon atoms; for example, R₁-G₁- may have 1, 2, 3, 4, 5, 6, 7, 8, 9, or 10 contiguous carbon atoms.
G₂ and G₃ are each independently selected from a chemical bond (-), C₁₋₁₂ alkylene, C₂₋₁₂ alkenylene, C₂₋₁₂ alkynylene, C₃₋₁₂ cycloalkylene, C₃₋₁₂ cycloalkenylene, C₃₋₁₂ cycloalkynylene, and C₆₋₁₂ arylene; L₁ and L₂ are each independently selected from one or a combination of two or more of a chemical bond (-), -O-, -S-, -O(C=O)O-, -(C=O)NRₐ-, -NRₐ(C=O)-, -O(C=O)-, -(C=O)O-, -S-S-, -S(O)ₓ-, - OS(O)ₓO-, -C(=O)S-, -SC(=O)-, -NRₐC(=O)NR_{b}-, -OC(=O)NRₐ-, -NRₐC(=O)O-, -OC(=O)S-, - SC(=O)O-, -P(O)(ORₐ)O-, -OP(O)(ORₐ)O-, and C₁₋₁₂ alkylene;
wherein x is selected from 0, 1, and 2;
wherein Rₐ and R_{b} are each independently selected from H, C₁₋₁₂ alkyl, C₂₋₁₂ alkenyl, and C₂₋₁₂ alkynyl; R₂ is selected from a naturally occurring or non-naturally occurring steroid;
R₃ is selected from a naturally occurring or non-naturally occurring steroid, C₆₋₂₄ alkyl, C₆₋₂₄ alkenyl, C₆₋₂₄ alkynyl, and C₆₋₂₄ alkoxy.

In one embodiment, R₁ is selected from -OR₄, -NR₄R₅, pyrazolyl, imidazolyl, piperazinyl, piperazinylalkyl, piperidinyl, piperidinylalkyl, guanidino, pyrrolyl, and pyrrolidinyl; R₄ and R₅ are each independently selected from H, C₁₋₉ alkyl, C₂₋₉ alkenyl, C₂₋₉ alkynyl, C₃₋₈ cycloalkyl, and C₃₋₈ cycloalkenyl;
G₁ is a chemical bond (-), C₁₋₉ alkylene, C₂₋₉ alkenylene, C₃₋₈ cycloalkylene, or C₃₋₈ cycloalkenylene; preferably, G₁ is C₁₋₆ alkylene or C₂₋₆ alkenylene;
R₁-G₁- has no more than 10 contiguous carbon atoms; specifically, R₁-G₁- may have 1, 2, 3, 4, 5, 6, 7, 8, 9, or 10 contiguous carbon atoms;
G₂ and G₃ are each independently selected from a chemical bond (-), C₁₋₁₂ alkylene, C₂₋₁₂ alkenylene, and C₂₋₁₂ alkynylene;
L₁ and L₂ are each independently selected from one or a combination of two or more of -O(C=O)-, - (C=O)O-, -S-S-, -O(C=O)O-, -NRₐC(=O)O-, -(C=O)NRₐ-, -NRₐ(C=O)-, -C(=O)S-, -SC(=O)-, - OC(=O)NRₐ-, and C₁₋₁₂ alkylene;
wherein Rₐ is H, C₁₋₁₂ alkyl, C₂₋₁₂ alkenyl, or C₂₋₁₂ alkynyl.
In one embodiment, R₁ is selected from -OR₄, -NR₄R₅, imidazolyl, piperazinyl, and guanidino;
R₄ and R₅ are each independently selected from H and -C₁₋₅ alkyl;
G₁ is unsubstituted C₁₋₆ alkylene;
R₁-G₁- has no more than 10 contiguous carbon atoms; specifically, R₁-G₁- may have 1, 2, 3, 4, 5, 6, 7, 8, 9, or 10 contiguous carbon atoms;
G₂ and G₃ are each independently selected from a chemical bond (-) and C₁₋₁₀ alkylene;
L₁ and L₂ are each independently selected from -O-, -O(C=O)-, -(C=O)O-, -(C=O)S-, -O(C=O)O-, - NHC(=O)O-, -NHC(=O)-, 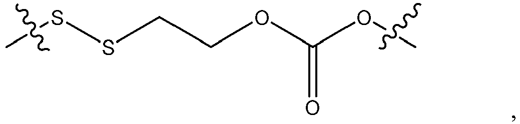 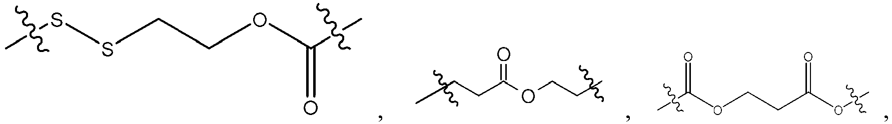 and

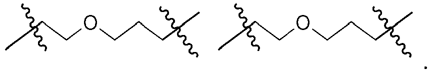

In one embodiment, R₂ is a sterol;
preferably, the sterol is a zoosterol or an oxidized or reduced form thereof; and/or the sterol is a phytosterol or an oxidized or reduced form thereof; and/or the sterol is a synthetic sterol or an oxidized or reduced form thereof;
more preferably, the sterol is selected from cholesterol, an oxidized form of cholesterol, a reduced form of cholesterol, alkyl lithocholate, stigmasterol, stigmastanol, campesterol, ergosterol, or/and sitosterol;
more preferably, the sterol is an oxidized form of cholesterol, a reduced form of cholesterol, alkyl lithocholate, stigmasterol, stigmastanol, campesterol, ergosterol, or/and sitosterol;
more preferably, the sterol is selected from avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, or/and lithocholic acid;
more preferably, the sterol has the following structural formula: 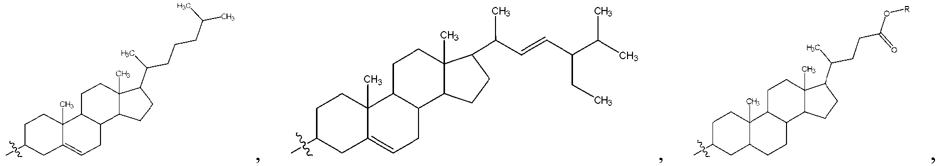 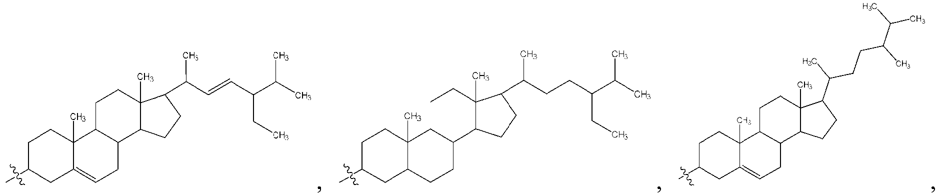 or 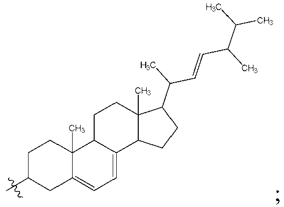
wherein R is C₁₋₂₀ alkyl.

In one embodiment, R₃ is selected from the following structures: 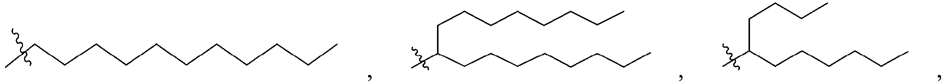 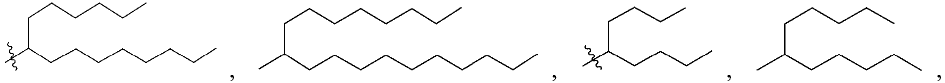 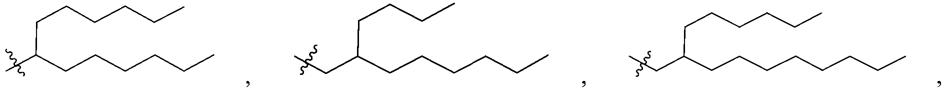 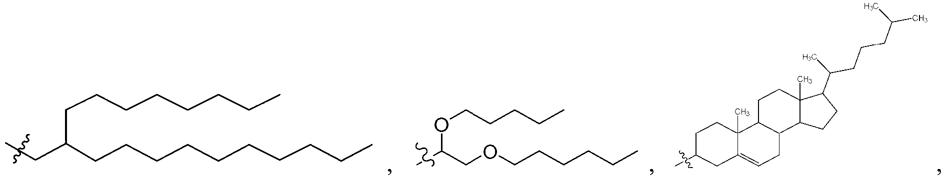 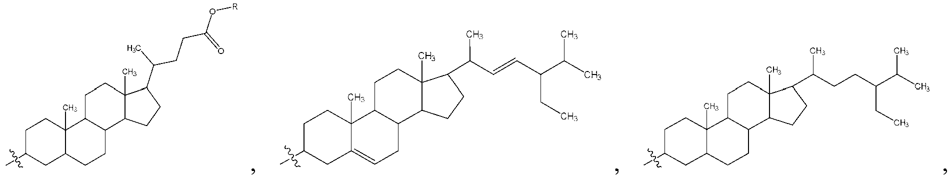 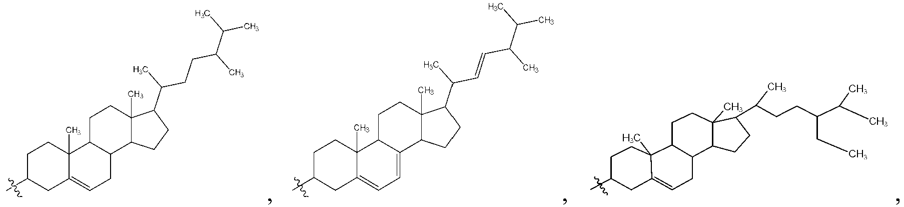 and

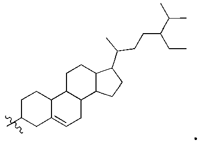

Preferably, R₃ is selected from the following structures:

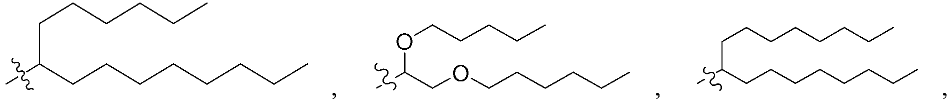

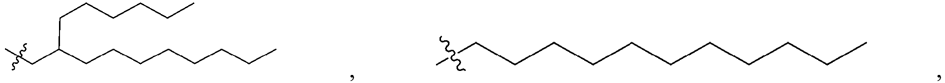

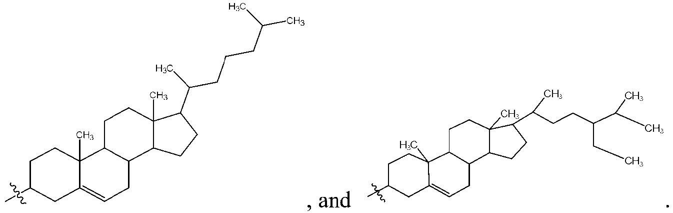

In one embodiment, the steroid-cationic lipid compound in the composition is selected from: 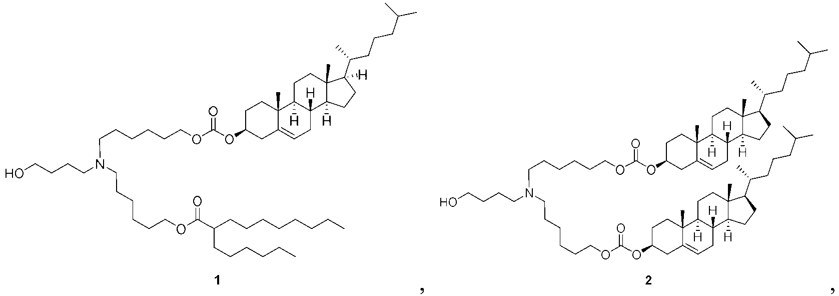 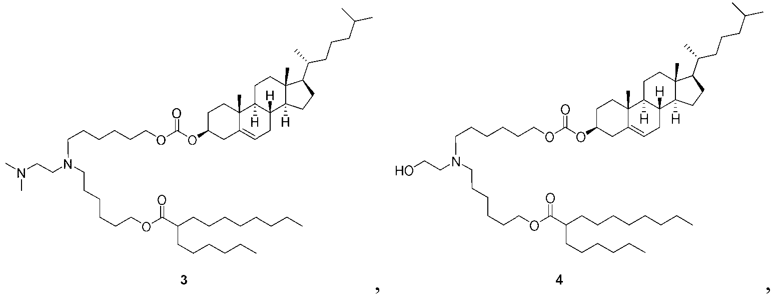 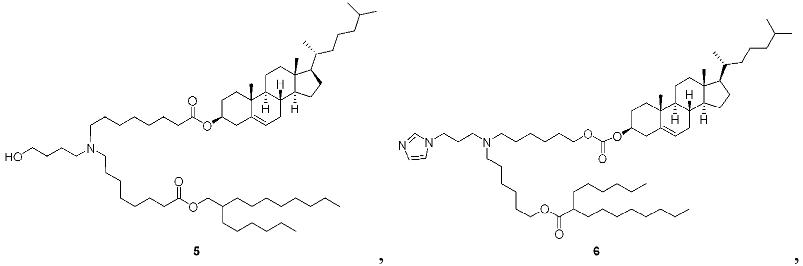 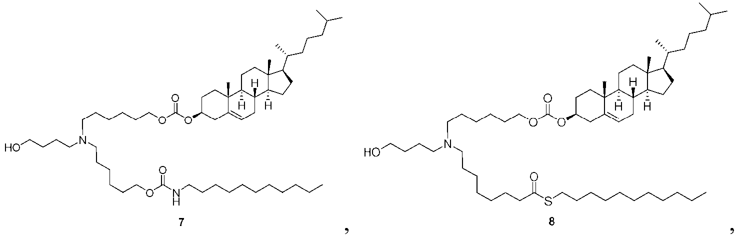 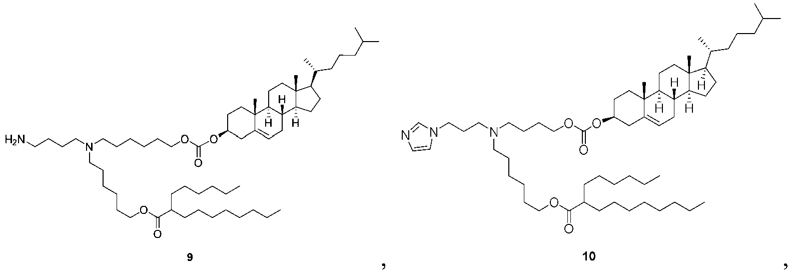 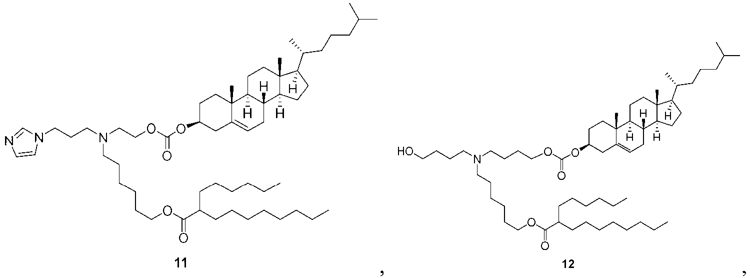 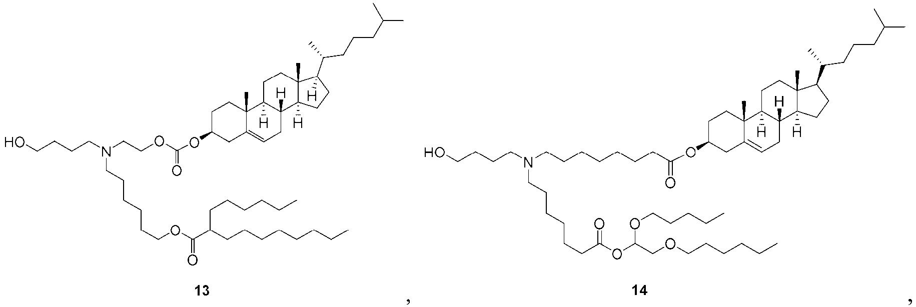 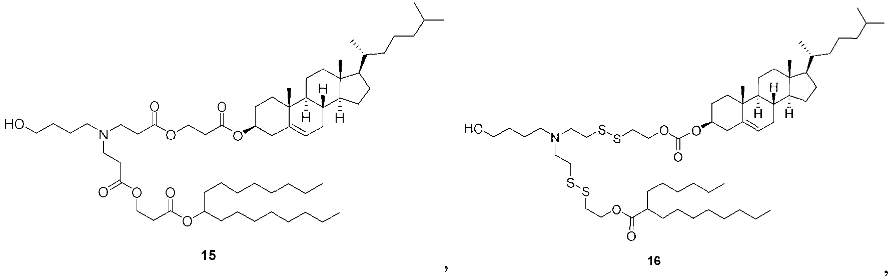 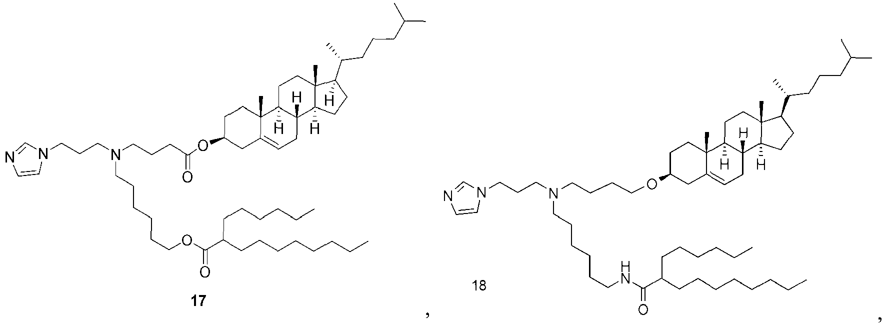 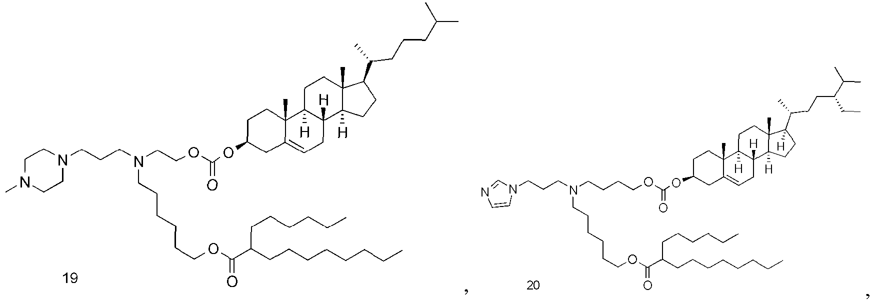 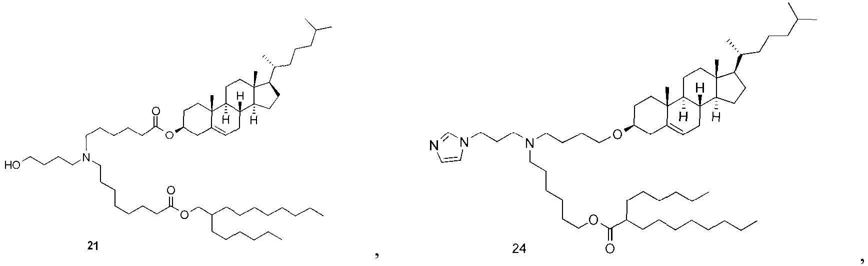 and

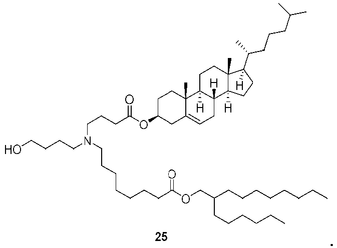

In one embodiment, a nitrogen-to-phosphorus ratio of the lipid nanoparticle is (1-15):1, including but not limited to, 1:1, 2:1, 3:1, 4:1, 5:1, 6:1, 7:1, 8:1, 9:1, 10:1, 11:1, 12:1, 13:1, 14:1, or 15:1.

In one embodiment, the lipid nanoparticle has a diameter of 15-300 nm; preferably, the lipid nanoparticle has a diameter of 60-102 nm; more preferably, the lipid nanoparticle has a diameter of 80-90 nm.

In one embodiment, a dosage form of the lipid nanoparticle composition is a liquid formulation or a lyophilized powder formulation.

In a second aspect, the present disclosure provides a preparation method for the lipid nanoparticle described herein, comprising dissolving the steroid-cationic lipid compound, the neutral phospholipid, and the polyethylene glycol lipid of the present disclosure in a solvent, and then mixing with a nucleic acid drug to give the lipid nanoparticle.

In one embodiment, the nucleic acid drug is an mRNA.

In a third aspect, the present disclosure provides use of the lipid nanoparticle composition described herein in preparing a bioactive substance delivery system.

In one embodiment, the bioactive substance delivery system is an aerosol inhalation delivery system. In one embodiment, a dosage form of the aerosol inhalation delivery system is a liquid formulation or a lyophilized powder formulation.

In one embodiment, the aerosol inhalation delivery system is administered by nebulizing a liquid formulation, and/or by nasal inhalation or oral inhalation; or the administration route of aerosol inhalation is transmucosal administration, and the liquid formulation is administered by aerosol inhalation. Preferably, the administration route of aerosol inhalation is nasal inhalation or oral inhalation to the mucosa.

In one embodiment, the aerosol inhalation delivery system is a liquid formulation, which can be administered by intramuscular injection.

In one embodiment, the delivery system further comprises a pharmaceutically acceptable excipient; preferably, the excipient comprises one or a combination of two or more of sodium acetate, tromethamine, potassium dihydrogen phosphate, sodium chloride, disodium hydrogen phosphate, and sucrose.

In one embodiment, the delivery system is a vaccine.

In one embodiment, the vaccine is a vaccine for preventing a cancer, a virus infection, a bacterial infection, or a fungal infection; preferably, the virus is selected from: norovirus, Ebola virus, coronavirus, cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, and measles virus.

The present disclosure has the following beneficial effects:
The lipid nanoparticle composition prepared by using the steroid-cationic lipid compound in the present disclosure has good stability and transfection efficiency. When the lipid nanoparticle is used to deliver a nucleic acid drug, such as mRNA, it can efficiently and stably deliver the nucleic acid drug to a target cell or organ, and meanwhile, the LNP can be used for aerosol inhalation administration of the mRNA. The lipid nanoparticle can induce a higher specific antibody response in an experimental animal while having better safety.

### BRIEF DESCRIPTION OF THE DRAWINGS

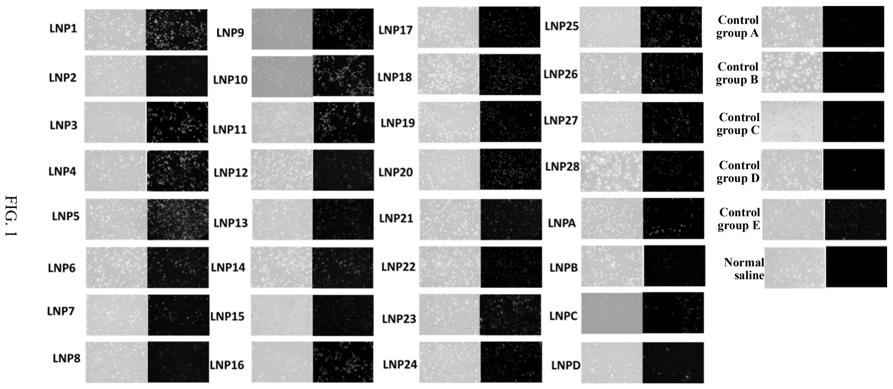

FIG. 1 shows the expression of GFP-mRNA-LNPs in Hep3B cells as detected by fluorescence microscopy;

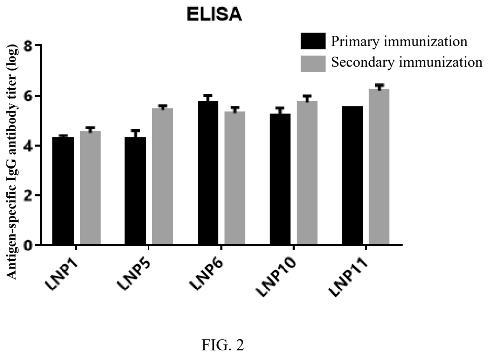

FIG. 2 shows the serum antibody titers, as detected by ELISA, after mice were immunized with mRNA-LNPs;

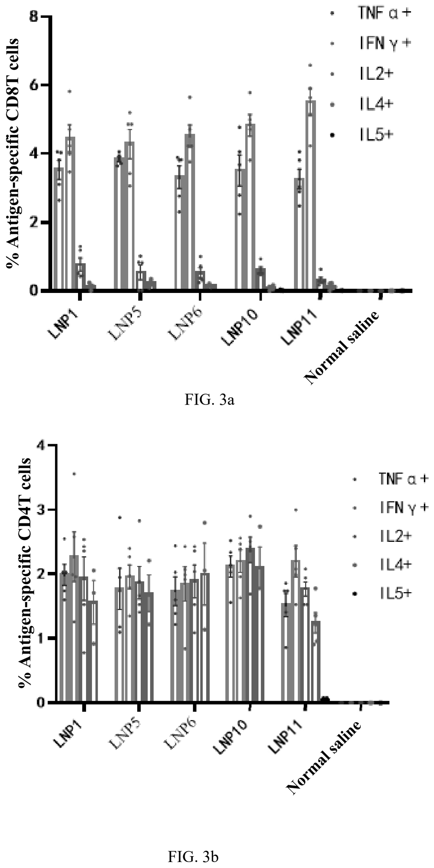

FIG. 3a shows the CD8T cell immunity, as detected by ICS, after mice were immunized with mRNA-LNPs;
FIG. 3b shows the CD4T cell immunity, as detected by ICS, after mice were immunized with mRNA-LNPs;

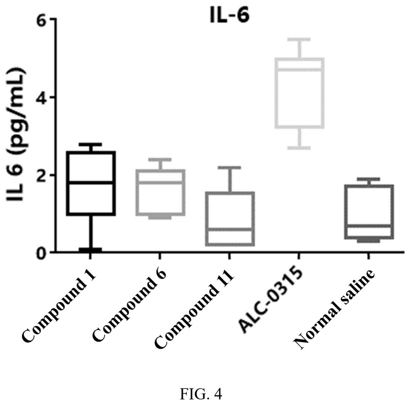

FIG. 4 shows the serum IL-6 expression in mice after high-dose administration of mRNA-LNPs;

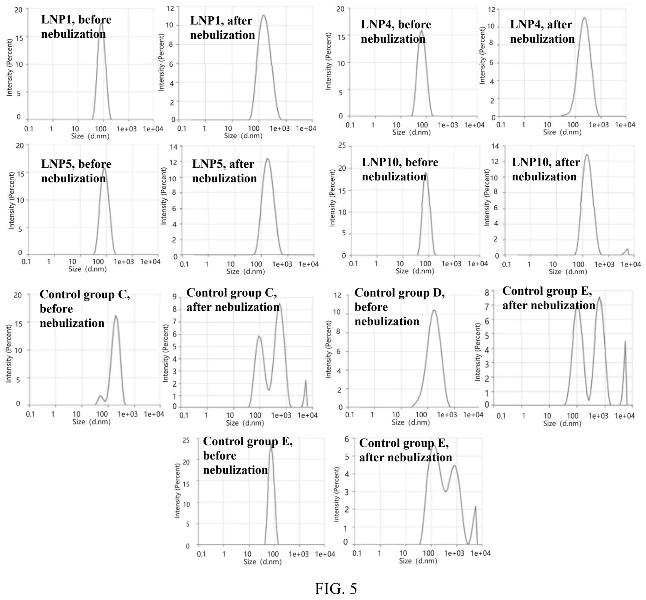

FIG. 5 shows the comparison of DLS before and after LNP nebulization;

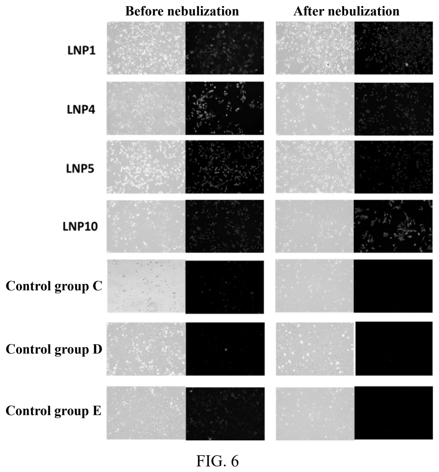

FIG. 6 shows the expression of GFP fluorescent protein after transfection of GFP-mRNA-LNPs into Hep3B cells before and after nebulization as detected by fluorescence microscopy;

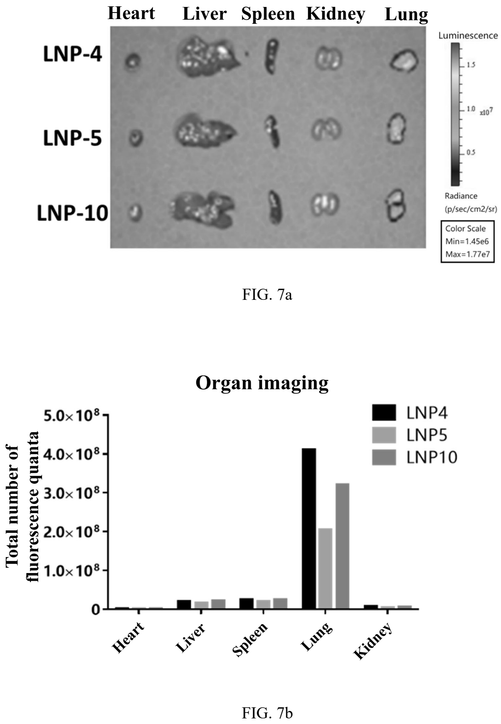

FIG. 7a shows the *in vivo* and organ images of mice after aerosol inhalation of Luc-mRNA-LNPs;
FIG. 7b shows the *in vivo* and organ imaging of mice after aerosol inhalation of Luc-mRNA-LNPs.

### DETAILED DESCRIPTION

The technical solutions in the present disclosure will be clearly and completely described below with reference to the drawings of the present disclosure. It is apparent that the described examples are only a part of the examples of the present disclosure, rather than all of them. It should be noted that the methods used in the present disclosure are all conventional methods unless otherwise specified, and the reagents used in the present disclosure are all commercially available products unless otherwise specified. Based on the examples of the present disclosure, all other examples obtained by those of ordinary skill in the art without creative work shall fall within the claimed scope of the present disclosure.

### Definitions

As used in this specification, the following words and phrases are generally intended to have the meanings as set forth below, unless the context in which they are used indicates otherwise.

As used herein, the term "lipid nanoparticle" or "LNP" refers to a particle with a nanoscale size (e.g., 1 nm to 1,000 nm) comprising one or more types of lipid molecules.

As used herein, the term "gene drug" generally consists of a carrier or a delivery system comprising an engineered gene construct. Its active ingredient may be a DNA, an RNA, or a genetically modified virus, bacterium, or cell. By introducing an exogenous gene into a target cell or tissue, a specific gene is replaced, compensated, blocked, or corrected, thereby achieving the purposes of treating and preventing diseases.

As used herein, the term "nucleic acid" refers to a polymer containing at least two kinds of deoxyribonucleotides or ribonucleotides in either single- or double-stranded form and includes a DNA, an RNA, and a hybrid thereof.

As used herein, the term "lipid compound" or "lipid" refers to a group of organic compounds that include, but are not limited to, esters of fatty acids and are generally characterized by being poorly soluble in water, but soluble in many organic solvents. The solvents of the present disclosure include, but are not limited to: water, benzene, toluene, pentane, hexane, methanol, ethanol, isopropanol, diethyl ether, ethyl acetate, acetone, and tetrachloromethane. In some embodiments of the present disclosure, the solvent is ethanol.

As used herein, the term "alkyl" refers to a saturated linear or branched hydrocarbon group. As used herein, C₁₋₉ alkyl includes linear or branched alkyl containing 1, 2, 3, 4, 5, 6, 7, 8, or 9 carbon atoms, for example, including but not limited to, C₁₋₈, C₂₋₇, C₂₋₈, C₃₋₆, or C₄₋₇ alkyl; C₆₋₂₄ alkyl includes linear or branched alkyl containing 6, 7, 8, 9, 10, 11, 12, 13, 14, 15, 16, 17, 18, 19, 20, 21, 22, 23, or 24 carbon atoms, for example, including but not limited to, C₆₋₈, C₆₋₁₇, C₆₋₂₂, C₁₀₋₁₆, or C₁₄₋₁₇ alkyl; C₁₋₅ alkyl includes linear or branched alkyl containing 1, 2, 3, 4, or 5 carbon atoms, for example, including but not limited to, C₁₋₄, C₂₋₄, C₂₋₃, C₃₋₅, or C₄₋₅ alkyl; C₁₋₂₀ alkyl includes linear or branched alkyl containing 1, 2, 3, 4, 5, 6, 7, 8, 9, 10, 11, 12, 13, 14, 15, 16, 17, 18, 19, or 20 carbon atoms, for example, including but not limited to, C₁₋₈, C₁₋₁₀, C₆₋₂₂, C₁₀₋₁₆, or C₁₄₋₁₇ alkyl.

As used herein, the term "alkenyl" refers to an unsaturated linear or branched hydrocarbon group containing one or more unsaturated carbon-carbon double bonds. The unsaturated carbon-carbon double bond may be present at any stable point along the chain. As used herein, C₂₋₉ alkenyl includes linear or branched alkenyl containing 2, 3, 4, 5, 6, 7, 8, or 9 carbon atoms, for example, including but not limited to, C₁₋₈, C₂₋₇, C₂₋₈, C₃₋₆, or C₄₋₈ linear or branched alkenyl; C₆₋₂₄ alkenyl includes linear or branched alkenyl containing 6, 7, 8, 9, 10, 11, 12, 13, 14, 15, 16, 17, 18, 19, 20, 21, 22, 23, or 24 carbon atoms, for example, including but not limited to, C₆₋₈, C₆₋₁₇, C₆₋₂₂, C₁₀₋₁₆, or C₁₄₋₁₇ alkenyl. As used herein, the term "alkynyl" refers to an unsaturated linear or branched hydrocarbon group containing one or more unsaturated carbon-carbon triple bonds. As used herein, C₂₋₉ alkynyl includes linear or branched alkynyl containing 2, 3, 4, 5, 6, 7, 8, or 9 carbon atoms, for example, including but not limited to, C₁₋₈, C₂₋₇, C₂₋₈, C₃₋₆, or C₄₋₇ alkynyl.

As used herein, one or more carbons at positions other than terminal positions of alkyl, alkenyl, and alkynyl may be substituted with heteroatoms such as nitrogen, oxygen, sulfur, and oxides thereof such as nitrogen oxides, carbonyl, a sulfone (sulfoxide) group; for example,-(CH₂)ₘ-O-(CH₂)ₙ-, -(CH₂)ₘ-S-(CH₂)ₙ-, -(CH₂)ₘ-S-S-(CH₂)ₙ-, -(CH₂)ₘ-CO-(CH₂)ₙ-, -(CH₂)ₘ-OCO-(CH₂)ₙ-, or -(CH₂)ₘ-OCOO-(CH₂)ₙ- (wherein m and n may be integers from 1 to 9) may be unsubstituted or substituted with one or more heteroatom substituents as described herein. In certain embodiments, the alkyl, alkenyl, or alkynyl does not contain any heteroatoms therein.

As used herein, the term "alkylene" refers to a saturated divalent linear or branched hydrocarbon group. As used herein, C₁₋₉ alkylene includes linear or branched alkylene containing 1, 2, 3, 4, 5, 6, 7, 8, or 9 carbon atoms, for example, including but not limited to, C₁₋₈, C₂₋₇, C₂₋₈, C₃₋₆, or C₄₋₇ alkylene; C₁₋₁₂ alkylene includes linear or branched alkylene containing 1, 2, 3, 4, 5, 6, 7, 8, 9, 10, 11, or 12 carbon atoms, for example, including but not limited to, C₁₋₈, C₂₋₇, C₂₋₈, C₃₋₆, or C₄₋₁₂ alkylene; C₁₋₁₀ alkylene includes linear or branched alkylene containing 1, 2, 3, 4, 5, 6, 7, 8, 9, or 10 carbon atoms, for example, including but not limited to, C₁₋₈, C₂₋₇, C₂₋₈, C₃₋₆, or C₄₋₁₀ alkylene; C₁₋₆ alkylene includes linear or branched alkylene containing 1, 2, 3, 4, 5, or 6 carbon atoms, for example, including but not limited to, C₁₋₅, C₂₋₄, C₃₋₆, or C₄₋₆ alkylene.

As used herein, the term "alkenylene" refers to an unsaturated linear or branched hydrocarbon group containing one or more unsaturated carbon-carbon double bonds. As used herein, C₂₋₉ alkenylene includes linear or branched alkenylene containing 2, 3, 4, 5, 6, 7, 8, or 9 carbon atoms, for example, including but not limited to, C₂₋₇, C₂₋₈, C₃₋₆, or C₄₋₇ alkenylene; C₂₋₁₂ alkenylene includes linear or branched alkenylene containing 2, 3, 4, 5, 6, 7, 8, 9, 10, 11, or 12 carbon atoms, for example, including but not limited to, C₂₋₈, C₂₋₇, C₃₋₆, or C₄₋₁₂ alkenylene; C₂₋₆ alkenylene includes linear or branched alkenylene containing 2, 3, 4, 5, or 6 carbon atoms, for example, including but not limited to, C₂₋₅, C₂₋₄, C₃₋₆, or C₄₋₆ alkenylene.

As used herein, the term "alkynylene" refers to an unsaturated linear or branched hydrocarbon group containing one or more unsaturated carbon-carbon triple bonds. As used herein, C₂₋₁₂ alkynylene includes linear or branched alkynylene containing 2, 3, 4, 5, 6, 7, 8, 9, 10, 11, or 12 carbon atoms, for example, including but not limited to, C₂₋₈, C₂₋₇, C₃₋₆, or C₄₋₁₂ alkynylene; C₃₋₉ alkynylene includes linear or branched alkynylene containing 3, 4, 5, 6, 7, 8, or 9 carbon atoms, for example, including but not limited to, C₃₋₆, C₃₋₈, or C₄₋₇ alkynylene.

As used herein, the alkylene, alkenylene, or alkynylene may contain one or more cyclic aliphatic groups and/or one or more heteroatoms such as oxygen, nitrogen, or sulfur, and may be optionally substituted with one or more substituents such as alkyl, halogen, alkoxy, hydroxy, amino, aryl, ether, ester, or amide. One or more carbons at positions other than terminal positions may be substituted with heteroatoms such as nitrogen, oxygen, sulfur, and oxides thereof such as nitrogen oxides, carbonyl, a sulfone or sulfoxide group. In certain embodiments, the alkylene, alkenylene, or alkynylene is unsubstituted. In certain embodiments, the alkylene, alkenylene, or alkynylene does not contain any heteroatoms.

As used herein, the term "nitrogen-containing heterocyclic group" is a heterocyclic group containing a nitrogen atom in the structure, including but not limited to, substituted or unsubstituted aziridinyl, azetidinyl, β-propiolactamyl, pyrrolyl, piperidinylalkyl, pyrazolyl, imidazolyl, oxazolyl, isoxazolyl, thiazolyl, pyridinyl, caprolactamyl, pyranyl, pyridazinyl, pyrimidinyl, pyrazinyl, piperazinyl, piperazinylalkyl, indolyl, benzimidazolyl, carbazolyl, quinolinyl, isoquinolinyl, pteridinyl, acridinyl, 7H-purinyl, phenazinyl, phenothiazinyl, or 1H-azepinyl.

As used herein, the term "alkoxy" refers to an "alkyl-O-" group, wherein the alkyl is as defined herein. As used herein, C₆₋₂₄ alkoxy includes linear or branched alkoxy containing 6, 7, 8, 9, 10, 11, 12, 13, 14, 15, 16, 17, 18, 19, 20, 21, 22, 23, or 24 carbon atoms, for example, including but not limited to, C₆₋₈, C₆₋₁₇, C₆₋₂₂, C₁₀₋₁₆, or C₁₄₋₁₇ alkoxy.

As used herein, the term "cycloalkyl" refers to a saturated cyclic hydrocarbon group. As used herein, C₃₋₈ cycloalkyl includes cycloalkyl containing 3, 4, 5, 6, 7, or 8 carbon atoms, for example, including but not limited to, C₃₋₇, C₄₋₇, or C₃₋₆ cycloalkyl.

As used herein, the term "cycloalkenyl" refers to a cyclic hydrocarbon group containing at least one carbon-carbon double bond. As used herein, C₃₋₈ cycloalkenyl includes cycloalkenyl containing 3, 4, 5, 6, 7, or 8 carbon atoms, for example, including but not limited to, C₃₋₇, C₄₋₇, or C₃₋₆ cycloalkenyl.

As used herein, the term "cycloalkynyl" refers to a cyclic hydrocarbon group containing at least one carbon-carbon triple bond. As used herein, C₃₋₈ cycloalkynyl includes cycloalkynyl containing 3, 4, 5, 6, 7, or 8 carbon atoms, for example, including but not limited to, C₃₋₇, C₄₋₇, or C₃₋₆ cycloalkynyl. As used herein, the term "cycloalkylene" refers to divalent cycloalkyl that is used to link two structures together. As used herein, C₃₋₁₂ cycloalkylene includes cycloalkylene containing 3, 4, 5, 6, 7, 8, 9, 10, 11, or 12 carbon atoms, for example, including but not limited to, C₃₋₈, C₄₋₇, C₅₋₈, or C₆₋₁₂ cycloalkylene; C₃₋₈ cycloalkylene includes cycloalkyl containing 3, 4, 5, 6, 7, or 8 carbon atoms, for example, including but not limited to, C₃₋₇, C₄₋₇, or C₆₋₈ cycloalkylene.

As used herein, the term "cycloalkenylene" refers to divalent cycloalkenyl that is used to link two structures together. As used herein, C₃₋₁₂ cycloalkenylene includes cycloalkenylene containing 3, 4, 5, 6, 7, 8, 9, 10, 11, or 12 carbon atoms, for example, including but not limited to, C₃₋₈, C₄₋₇, C₅₋₈, or C₆₋₁₂ cycloalkenylene; C₃₋₈ cycloalkenylene includes cycloalkenylene containing 3, 4, 5, 6, 7, or 8 carbon atoms, for example, including but not limited to, C₃₋₇, C₄₋₇, or C₆₋₈ cycloalkenylene.

As used herein, the term "cycloalkynylene" refers to divalent cycloalkynyl that is used to link two structures together. As used herein, C₃₋₁₂ cycloalkynylene includes cycloalkynylene containing 3, 4, 5, 6, 7, 8, 9, 10, 11, or 12 carbon atoms, for example, including but not limited to, C₃₋₈, C₄₋₇, C₅₋₈, or C₆₋₁₂ cycloalkynylene; C₃₋₈ cycloalkynylene includes cycloalkynylene containing 3, 4, 5, 6, 7, or 8 carbon atoms, for example, including but not limited to, C₃₋₇, C₄₋₇, or C₆₋₈ cycloalkynylene.

As used herein, the term "arylene" refers to divalent aryl that is used to link two structures together, wherein the aryl includes monocyclic aromatic (having, for example, 4n+2 delocalized electrons) groups and polycyclic aromatic groups. As used herein, C₆₋₁₂ arylene includes arylene containing 6, 7, 8, 9, 10, 11, or 12 carbon atoms, for example, including but not limited to, C₆₋₈, C₆₋₉, or C₆₋₁₂ arylene. As used herein, the term "cationic lipid" refers to a lipid molecule that is positively charged in response to ambient pH or hydrogen ion activity.

As used herein, the term "helper lipid" refers to a lipid that is not positively charged at ambient pH, including neutral lipids that are not charged at all, zwitterionic lipids, and anionic lipids that are negatively charged.

As used herein, the term "polyethylene glycol lipid" refers to a lipid molecule comprising a lipid portion and a polyethylene glycol portion.

As used herein, the term "delivery system" refers to a formulation or composition that regulates the spatial, temporal, and dose distribution of a bioactive ingredient in an organism.

### Examples

### Example 1. Synthesis of Compound 1

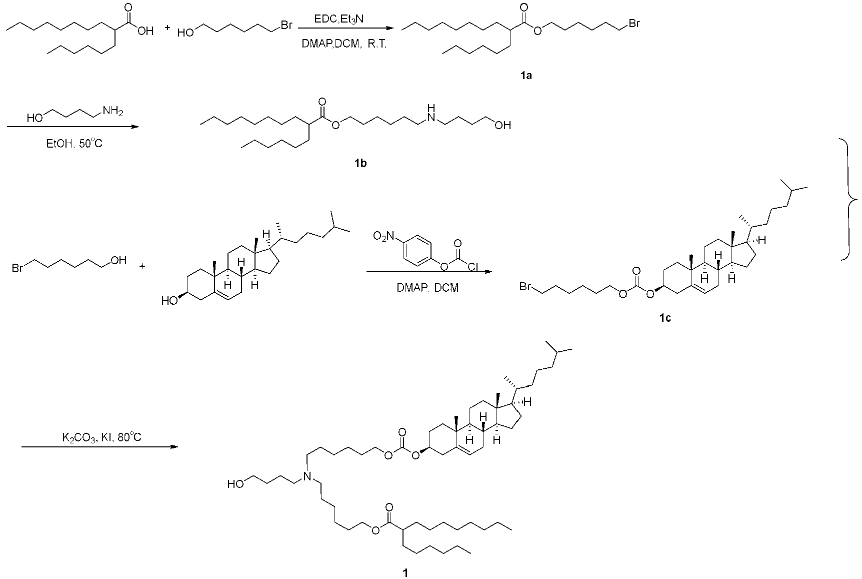

### Step 1: Synthesis of 6-bromohexyl 2-hexyldecanoate (1a)

2-Hexyldecanoic acid (2.12 g, 5.0 mmol) was dissolved in dichloromethane (30 mL), and 6-bromohexanol (0.93 g, 5.0 mmol), 4-dimethylaminopyridine (DMAP, 0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 1a (1.45 g, light yellow oil, yield: 70%).

MS m/z (ESI): 419.2 [M+1]

### Step 2: Synthesis of 6-((4-hydroxybutyl)amino)hexyl 2-hexyldecanoate (1b)

At room temperature, 6-bromohexyl 2-hexyldecanoate 1a (1.28 g, 3 mmol) was dissolved in ethanol (20 mL), and 4-amino-1-butanol (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 1b (1.13 g, light yellow oil).

MS m/z (ESI): 428.4 [M+1]

### Step 3: Synthesis of 6-bromohexyl cholesterol carbonate (1c)

6-Bromohexanol (0.91 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Cholesterol (2.16 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 1c (1.83 g, light yellow oil, yield: 62%).

MS m/z (ESI): 593.3 [M+1]

### Step 4: Synthesis of compound 1

6-((4-Hydroxybutyl)amino)hexyl 2-hexyldecanoate 1b (428 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 1 (470 mg, light yellow oil, yield: 50%).
MS m/z (ESI): 941.4 [M+1]
1H NMR (300MHz, CDCl3): δ 5.40 (t, 1H, J = 5.4 Hz), 4.53-4.40 (m, 1H), 4.20-4.02 (m, 4H), 3.67-3.61 (m, 2H), 3.58-3.50 (m, 2H), 2.47-2.42 (m, 8H), 2.03-1.72 (m, 5H), 1.67-0.85 (m, 82H), 0.70 (s, 3H)

### Example 2. Synthesis of Compound 2

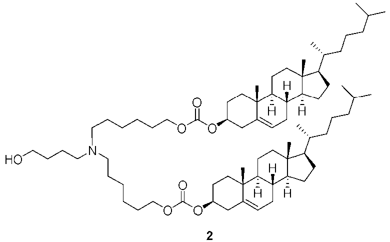

6-Bromohexyl cholesterol carbonate 1c (1.48 g, 2.5 mmol) was dissolved in tetrahydrofuran, and acetonitrile, 4-amino-1-butanol (89.2 mg, 1.0 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 2 (679 mg, light yellow solid, yield: 61%).
MS m/z (ESI): 1115.2 [M+1]
1H NMR (300MHz, CDCl₃): δ 5.40 (t, 2H, J = 5.4 Hz), 4.53-4.40 (m, 2H), 4.15 (t, 4H, J = 5.6 Hz), 3.76 (t, 2H, J = 5.4 Hz), 3.20-3.01 (m, 6H), 2.45-2.31 (m, 4H), 2.02-1.75 (m, 16H), 1.74-1.22 (m, 36H), 1.19-0.87 (m, 44H), 0.70 (s, 6H)

### Example 3. Synthesis of Compound 3

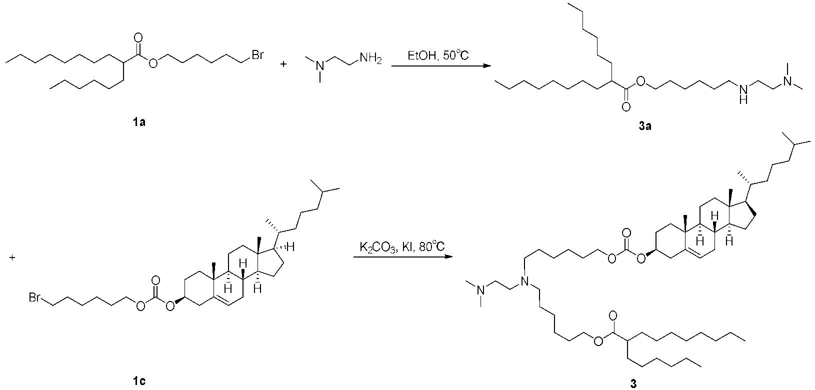

### Step 1: Synthesis of 6-((2-(dimethylamino)ethyl)amino)hexyl 2-hexyldecanoate (3a)

At room temperature, 6-bromohexyl 2-hexyldecanoate 1a (1.28 g, 3 mmol) was dissolved in ethanol (20 mL), and N,N-dimethylethylenediamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 3a (1.20 g, light yellow oil).

MS m/z (ESI): 427.4 [M+1]

### Step 2: Synthesis of compound 3

6-((2-(Dimethylamino)ethyl)amino)hexyl 2-hexyldecanoate 3a (426 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 3 (517 mg, light yellow oil, yield: 55%).
MS m/z (ESI): 940.0 [M+1]
1H NMR (300MHz, CDCl3): δ 5.40 (t, 1H, J = 5.4 Hz), 4.53-4.40 (m, 1H), 4.20-4.02 (m, 3H), 3.76-3.61 (m, 2H), 3.45-3.38 (m, 3H), 2.94-2.85 (m, 1H), 2.55-2.27 (m, 5H), 2.03-0.85 (m, 70H), 0.70 (s, 3H)

### Example 4. Synthesis of Compound 4

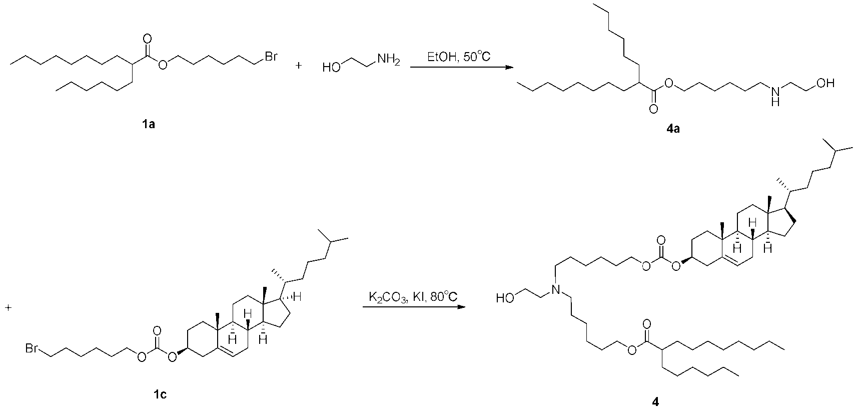

### Step 1: Synthesis of 6-((2-hydroxyethyl)amino)hexyl 2-hexyldecanoate (4a)

At room temperature, 6-bromohexyl 2-hexyldecanoate 1a (1.28 g, 3 mmol) was dissolved in ethanol (20 mL), and ethanolamine (2.75 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 4a (1.01 g, light yellow oil).

MS m/z (ESI): 400.4 [M+1]

### Step 2: Synthesis of compound 4

6-((2-Hydroxyethyl)amino)hexyl 2-hexyldecanoate 4a (400 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 4 (474 mg, light yellow oil, yield: 52%).
MS m/z (ESI): 913.1 [M+1]
1H NMR (300MHz, CDCl3): δ 5.40 (t, 1H, J = 5.4 Hz), 4.53-4.40 (m, 1H), 4.20-4.02 (m, 4H), 3.67-3.61 (m, 2H), 3.58-3.50 (m, 4H), 2.47-2.42 (m, 2H), 2.03-1.72 (m, 5H), 1.67-0.85 (m, 82H), 0.70 (s, 3H)

### Example 5. Synthesis of Compound 5

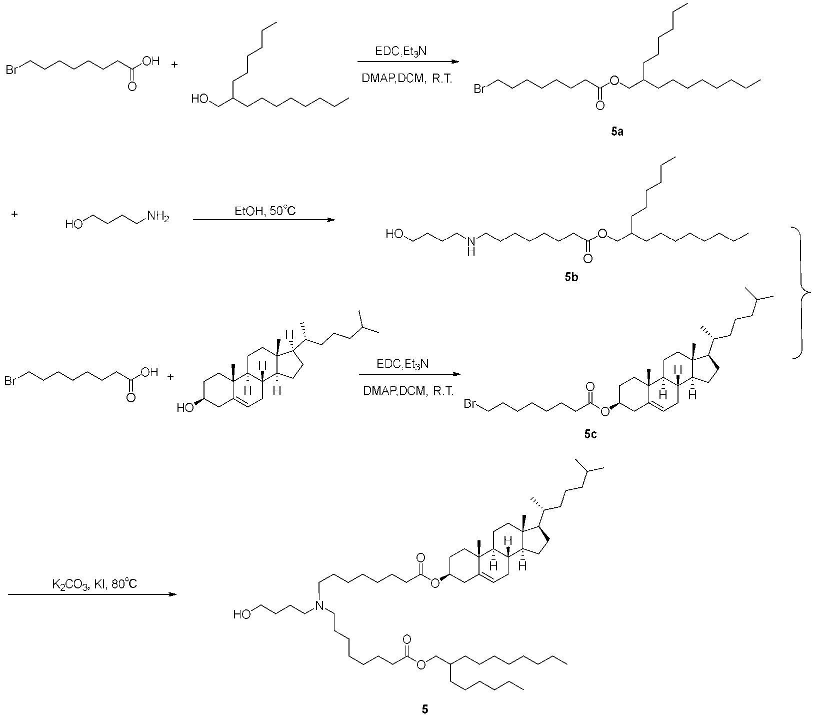

### Step 1: Synthesis of 2-hexyldecyl 8-bromooctanoate (5a)

8-Bromooctanoic acid (1.12 g, 5.0 mmol) was dissolved in dichloromethane (30 mL), and 2-hexyldecanol (1.21 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 5a (1.52 g, light yellow oil, yield: 68%).

MS m/z (ESI): 447.2 [M+1]

### Step 2: Synthesis of 2-hexyldecyl 8-((4-hydroxybutyl)amino)octanoate (5b)

At room temperature, 2-hexyldecyl 8-bromooctanoate 5a (1.34 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 5b (1.21 g, light yellow oil).

MS m/z (ESI): 456.4 [M+1]

### Step 3: Synthesis of cholesterol 8-bromooctanoate (5c)

8-Bromooctanoic acid (1.12 g, 5.0 mmol) was dissolved in dichloromethane (30 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 5c (1.77 g, light yellow oil, yield: 60%).

MS m/z (ESI): 591.4 [M+1]

### Step 4: Synthesis of compound 5

2-Hexyldecyl 8-((4-hydroxybutyl)amino)octanoate 5b (455 mg, 1.0 mmol) was dissolved in tetrahydrofuran, and acetonitrile, cholesterol 8-bromooctanoate 5c (708 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 5 (464 mg, light yellow oil, yield: 48%).
MS m/z (ESI): 967.6 [M+1]
1H NMR (300MHz, CDCl3): δ 5.40 (t, 1H, J = 5.4 Hz), 4.53-4.40 (m, 1H), 4.20-4.02 (m, 2H), 3.67-3.61 (m, 2H), 2.64 (t, 6H, J = 5.4 Hz), 2.37-2.25 (m, 6H), 2.10-0.85 (m, 93H), 0.70 (s, 3H)

### Example 6. Synthesis of Compound 6

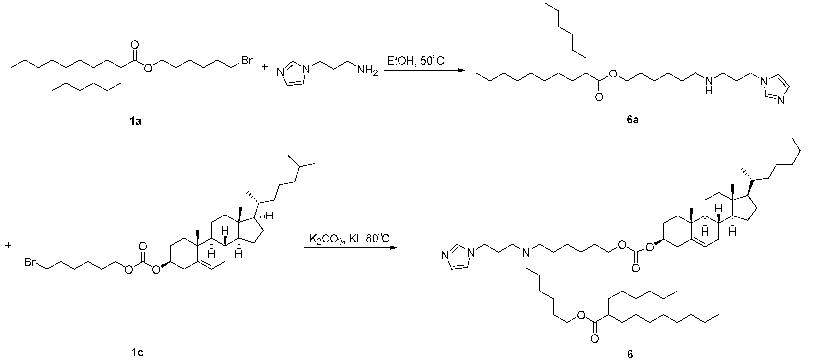

### Step 1: Synthesis of 6-((3-(1H-imidazol-1-yl)propyl)amino)hexyl 2-hexyldecanoate (6a)

At room temperature, 6-bromohexyl 2-hexyldecanoate 1a (1.28 g, 3 mmol) was dissolved in ethanol (20 mL), and 1-(3-aminopropyl)imidazole (5.63 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 6a (1.46 g, light yellow oil).

MS m/z (ESI): 464.4 [M+1]

### Step 2: Synthesis of compound 6

6-((3-(1H-Imidazol-1-yl)propyl)amino)hexyl 2-hexyldecanoate 6a (463 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 6 (517 mg, light yellow oil, yield: 53%).
MS m/z (ESI): 976.9 [M+1]
1H NMR (300MHz, CDCl3): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 5H), 2.47-2.30 (m, 9H), 2.36-0.85 (m, 87H), 0.70 (s, 3H)

### Example 7. Synthesis of Compound 7

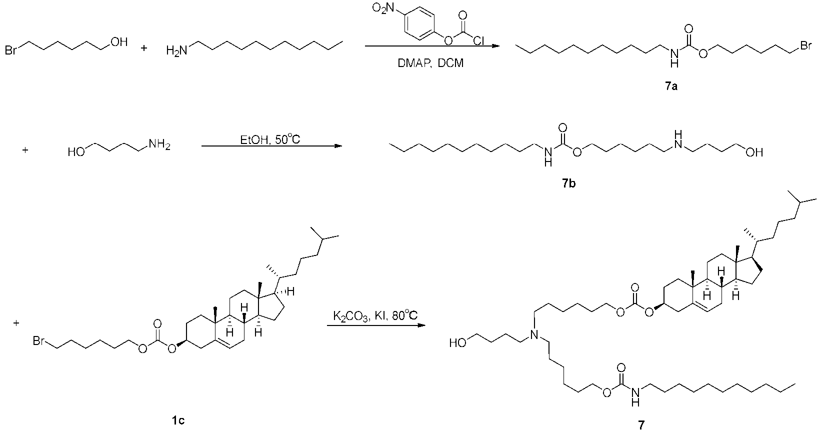

### Step 1: Synthesis of 6-bromohexyl undecylcarbamate (7a)

6-Bromohexanol (0.91 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Undecylamine (0.96 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give compound 7a (1.23 g, light yellow oil, yield: 65%).

MS m/z (ESI): 378.2 [M+1]

### Step 2: Synthesis of 6-((4-hydroxybutyl)amino)hexyl undecylcarbamate (7b)

At room temperature, 6-bromohexyl undecylcarbamate 7a (1.13 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 7b (1.14 g, light yellow oil).

MS m/z (ESI): 387.4 [M+1]

### Step 3: Synthesis of compound 7

6-((4-Hydroxybutyl)amino)hexyl undecylcarbamate 7b (386 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 7 (459 mg, light yellow oil, yield: 51%).
MS m/z (ESI): 900.4 [M+1]
1H NMR (300MHz, CDCl3): δ 5.42 (t, 1H, J = 5.4 Hz), 4.80-4.71 (m, 1H), 4.51-4.40 (m, 1H), 4.20-4.02 (m, 4H), 3.75-3.61 (m, 2H), 3.20-3.11 (m, 2H), 2.91-2.73 (m, 5H), 2.43-2.36 (m, 2H), 2.10-0.83 (m, 79H), 0.70 (s, 3H)

### Example 8. Synthesis of Compound 8

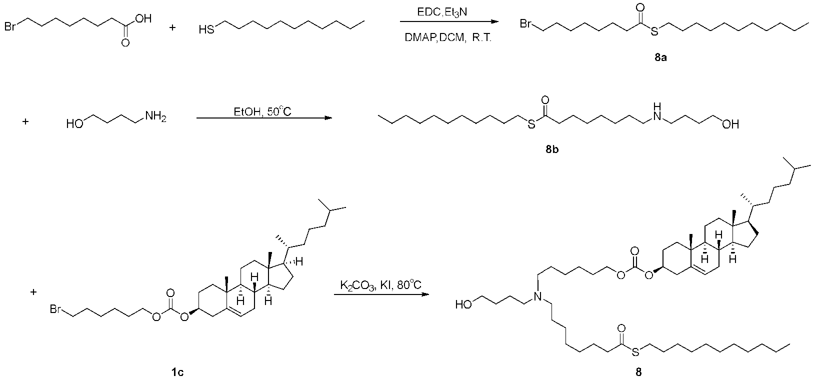

### Step 1: Synthesis of S-undecyl 8-bromooctanethioate (8a)

8-Bromooctanoic acid (1.12 g, 5.0 mmol) was dissolved in dichloromethane (30 mL), and undecanethiol (0.94 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 8a (1.30 g, light yellow oil, yield: 66%).

MS m/z (ESI): 393.2 [M+1]

### Step 2: Synthesis of S-undecyl 8-((4-hydroxybutyl)amino)octanethioate (8b)

At room temperature, S-undecyl 8-bromooctanethioate 8a (1.18 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 8b (1.18 g, light yellow oil).

MS m/z (ESI): 402.3 [M+1]

### Step 3: Synthesis of compound 8

S-Undecyl 8-((4-hydroxybutyl)amino)octanethioate 8b (401 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 8 (458 mg, light yellow oil, yield: 50%).
MS m/z (ESI): 914.9 [M+1]
1H NMR (300MHz, CDCl3): δ 5.42 (t, 1H, J = 5.4 Hz), 4.51-4.40 (m, 1H), 4.20-4.02 (m, 2H), 3.65-3.51 (m, 2H), 2.91-2.83 (m, 2H), 2.60-2.36 (m, 10H), 2.10-0.86 (m, 82H), 0.70 (s, 3H)

### Example 9. Synthesis of Compound 9

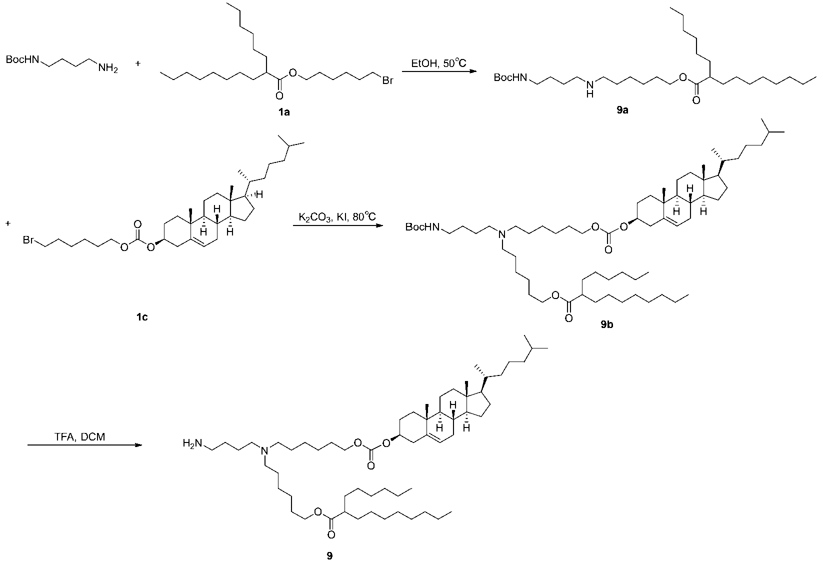

### Step 1: Synthesis of 6-((4-((tert-butoxycarbonyl)amino)butyl)amino)hexyl 2-hexyldecanoate (9a)

At room temperature, 6-bromohexyl 2-hexyldecanoate 1a (1.28 g, 3 mmol) was dissolved in ethanol (20 mL), and BOC-1,4-butanediamine hydrochloride (10.1 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 9a (1.51 g, light yellow oil).

MS m/z (ESI): 527.5 [M+1]

### Step 2: Synthesis of compound 9b

6-((4-((tert-Butoxycarbonyl)amino)butyl)amino)hexyl 2-hexyldecanoate 9a (526 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 9b (540 mg, light yellow oil, yield: 52%).

MS m/z (ESI): 1039.9 [M+1]

### Step 3: Synthesis of compound 9

Compound 9b (1.04 g, 1.0 mmol) was dissolved in dichloromethane, and a solution of trifluoroacetic acid in dichloromethane was added in an ice bath. The mixture was stirred at room temperature for 16 h. Trifluoroacetic acid was removed by rotary evaporation, and dichloromethane was added for dissolution. The resulting mixture was washed twice with a saturated sodium bicarbonate solution. The organic phase was dried over anhydrous sodium sulfate, filtered, and concentrated, and the residue was separated by column chromatography to give compound 9 (460 mg, light yellow oil, yield: 49%).
MS m/z (ESI): 940.1 [M+1]
1H NMR (300MHz, CDCl3): δ 5.40 (t, 1H, J = 5.4 Hz), 4.63-4.40 (m, 1H), 4.19-4.03 (m, 4H), 2.79-2.70 (m, 2H), 2.55-2.28 (m, 10H), 2.02-0.87 (m, 89H), 0.70 (s, 3H)

### Example 10. Synthesis of Compound 10

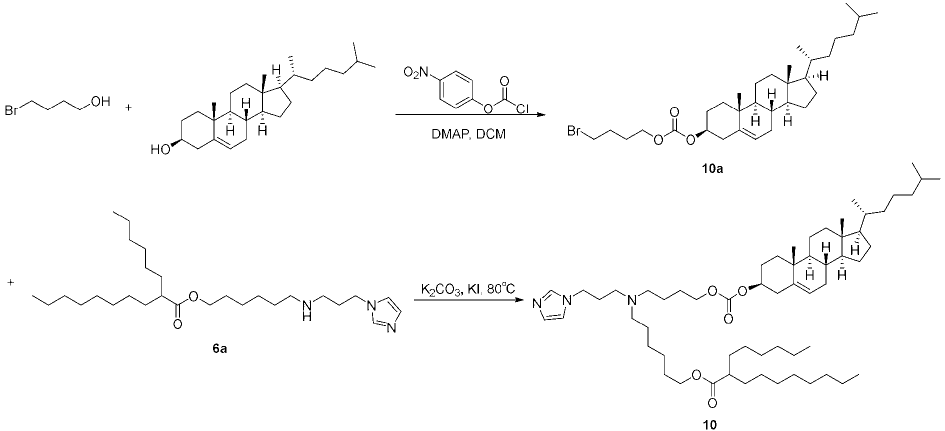

### Step 1: Synthesis of 4-bromobutyl cholesterol carbonate (10a)

4-Bromobutanol (0.77 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Cholesterol (2.16 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 10a (1.50 g, light yellow oil, yield: 53%).

MS m/z (ESI): 565.3 [M+1]

### Step 2: Synthesis of compound 10

6-((3-(1H-Imidazol-1-yl)propyl)amino)hexyl 2-hexyldecanoate 6a (463 mg, 1.0 mmol) was dissolved in tetrahydrofuran, and acetonitrile, 4-bromobutyl cholesterol carbonate 10a (678 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 10 (455 mg, light yellow oil, yield: 48%).
MS m/z (ESI): 948.9 [M+1]
1H NMR (300MHz, CDCl3): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 6H), 2.47-2.30 (m, 8H), 2.36-0.85 (m, 83H), 0.70 (s, 3H)

### Example 11. Synthesis of Compound 11

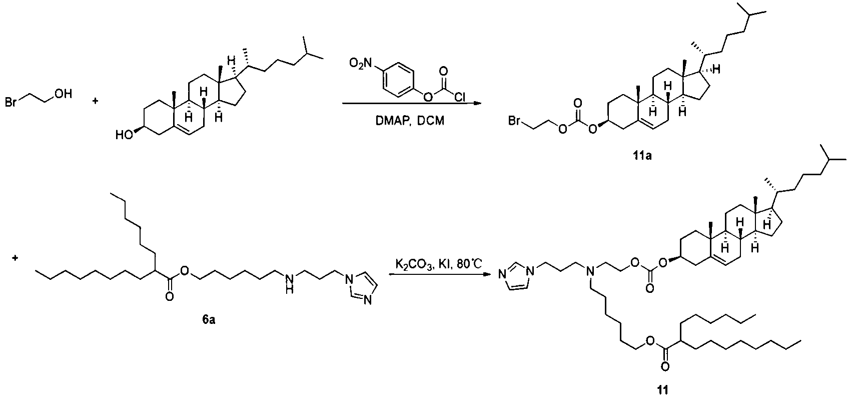

### Step 1: Synthesis of 2-bromoethyl cholesterol carbonate (11a)

2-Bromoethanol (0.63 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Cholesterol (2.16 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 11a (1.53 g, light yellow oil, yield: 57%).

MS m/z (ESI): 537.3 [M+1]

### Step 2: Synthesis of compound 11

6-((3-(1H-Imidazol-1-yl)propyl)amino)hexyl 2-hexyldecanoate 6a (463 mg, 1.0 mmol) was dissolved in tetrahydrofuran, and acetonitrile, 2-bromoethyl cholesterol carbonate 11a (644 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 11 (506 mg, light yellow oil, yield: 55%).
MS m/z (ESI): 920.8 [M+1]
1H NMR (300MHz, CDCl3): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 6H), 2.74-2.365 (m, 2H), 2.47-2.30 (m, 6H), 2.36-0.85 (m, 79H), 0.70 (s, 3H)

### Example 12. Synthesis of Compound 12

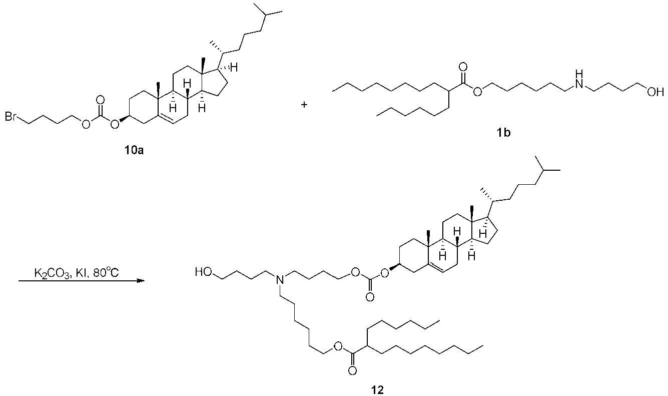

6-((4-Hydroxybutyl)amino)hexyl 2-hexyldecanoate 1b (428 mg, 1.0 mmol) was dissolved in tetrahydrofuran, and acetonitrile, 4-bromobutyl cholesterol carbonate 10a (679 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product 12 (566 mg, light yellow oil, yield: 62%).
MS m/z (ESI): 913.0 [M+1]
1H NMR (300MHz, CDCl3): δ 5.40 (t, 1H, J = 5.4 Hz), 4.53-4.40 (m, 1H), 4.20-4.02 (m, 4H), 3.67-3.61 (m, 2H), 2.63-2.32 (m, 8H), 2.03-1.72 (m, 5H), 1.67-0.85 (m, 80H), 0.70 (s, 3H)

### Example 13. Synthesis of Compound 13

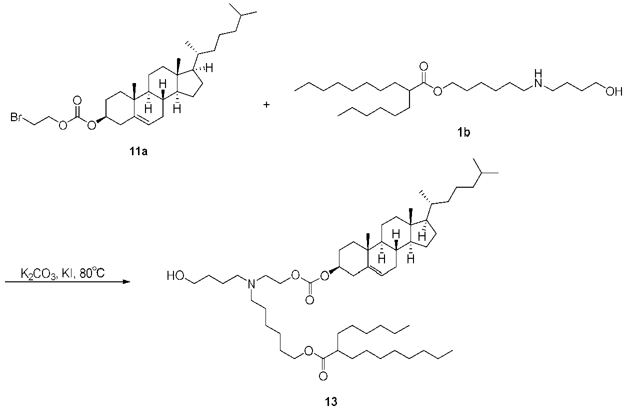

6-((4-Hydroxybutyl)amino)hexyl 2-hexyldecanoate 1b (428 mg, 1.0 mmol) was dissolved in tetrahydrofuran, and acetonitrile, 4-bromobutyl cholesterol carbonate 11a (645 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product 13 (451 mg, light yellow oil, yield: 51%).
MS m/z (ESI): 885.1 [M+1]
1H NMR (300MHz, CDCl₃): δ 5.40 (t, 1H, J = 5.4 Hz), 4.53-4.40 (m, 1H), 4.29-4.21 (m, 2H), 4.20-4.02 (m, 2H), 3.67-3.61 (m, 2H), 2.85-2.78 (m, 2H), 2.63-2.32 (m, 6H), 2.03-1.72 (m, 5H), 1.67-0.85 (m, 76H), 0.70 (s, 3H)

### Example 14. Synthesis of Compound 14

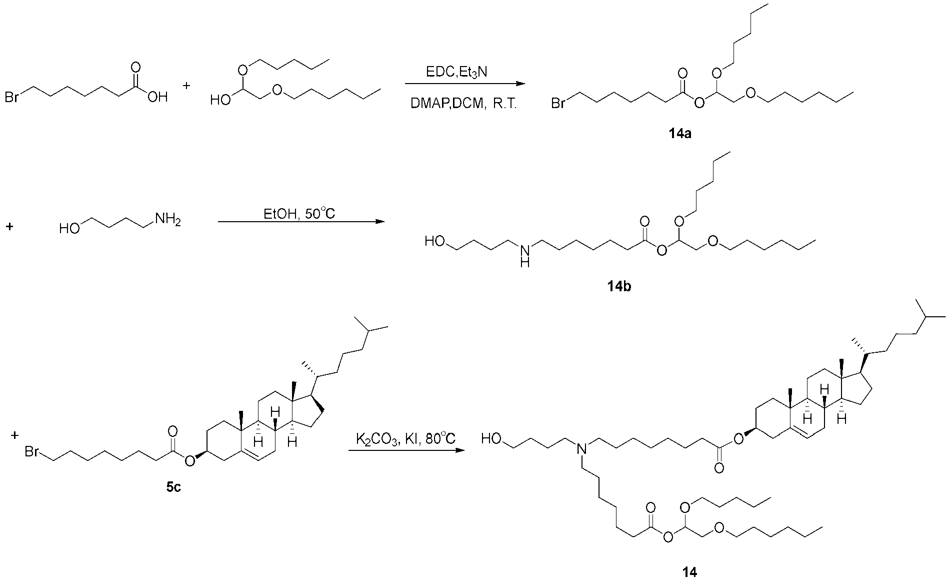

### Step 1: Synthesis of 2-(hexyloxy)-1-(pentyloxy)ethyl 7-bromoheptanoate (14a)

7-Bromoheptanoic acid (1.05 g, 5.0 mmol) was dissolved in dichloromethane (30 mL), and 2-(hexyloxy)-1-(pentyloxy)-1-ethanol (1.16 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 14a (1.08 g, light yellow oil, yield: 51%).

MS m/z (ESI): 423.2 [M+1]

### Step 2: Synthesis of 2-(hexyloxy)-1-(pentyloxy)ethyl 7-((4-hydroxybutyl)amino)heptanoate (14b)

At room temperature, 2-(hexyloxy)-1-(pentyloxy)ethyl 7-bromoheptanoate 14a (1.27 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 14b (1.05 g, light yellow oil).

MS m/z (ESI): 432.4 [M+1]

### Step 3: Synthesis of compound 14

2-(Hexyloxy)-1-(pentyloxy)ethyl 7-((4-hydroxybutyl)amino)heptanoate 14b (432 mg, 1.0 mmol) was dissolved in tetrahydrofuran, and acetonitrile, cholesterol 8-bromooctanoate 5c (708 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 14 (490 mg, light yellow oil, yield: 52%).
MS m/z (ESI): 942.8 [M+1]
1H NMR (300MHz, CDCl3): δ 6.67 (t, 1H, J= 6.3 Hz), 5.40 (t, 1H, J = 5.4 Hz), 4.53-4.40 (m, 1H), 4.20-4.02 (m, 2H), 3.67-3.61 (m, 2H), 3.47-3.31 (m, 4H), 2.64 (t, 6H, J = 5.4 Hz), 2.37-2.25 (m, 6H), 2.10-0.85 (m, 80H), 0.70 (s, 3H)

### Example 15. Synthesis of Compound 15

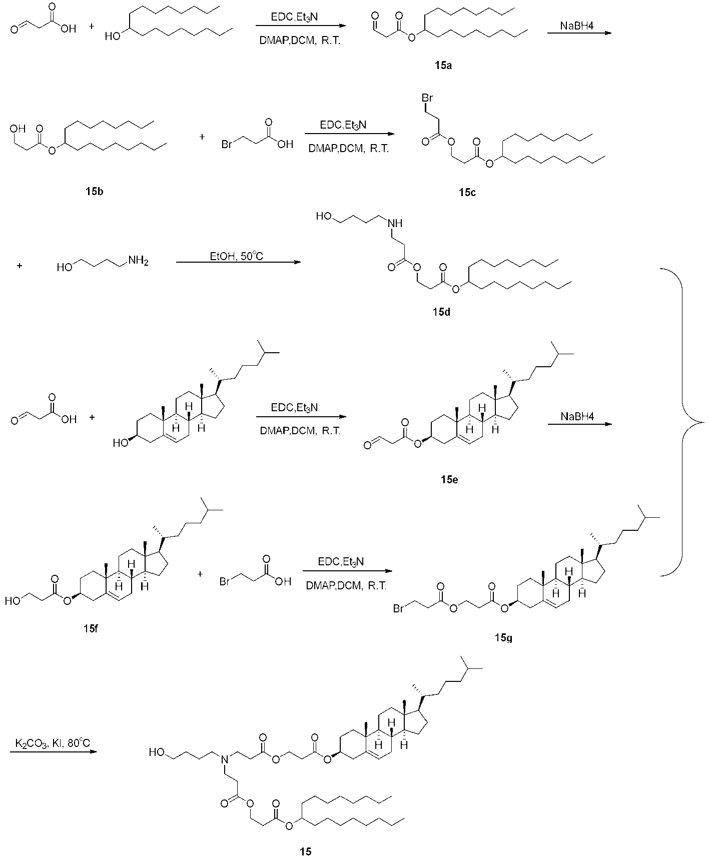

### Step 1: Synthesis of heptadecan-9-yl 3-oxopropanoate (15a)

3-Oxopropanoic acid (0.44 g, 5.0 mmol) was dissolved in dichloromethane (20 mL), and 9-heptadecanol (1.28 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15a (0.78 g, light yellow oil, yield: 48%).

MS m/z (ESI): 327.3 [M+1]

### Step 2: Synthesis of heptadecan-9-yl 3-hydroxypropanoate (15b)

Heptadecan-9-yl 3-oxopropanoate 15a (0.78 g, 2.3 mmol) was dissolved in n-butanol (10 mL), and sodium borohydride (266 mg, 7 mmol) was added at 0 °C. The mixture was stirred at room temperature for 12 h. After the starting materials were consumed completely as monitored, a saturated ammonium chloride solution was added to quench the reaction, and the resulting mixture was extracted with dichloromethane. The organic phase was dried over anhydrous sodium sulfate and concentrated to give compound 15b (0.70 g, light yellow oil).

MS m/z (ESI): 329.3 [M+1]

### Step 3: Synthesis of compound 15c

3-Bromopropanoic acid (306 mg, 2.0 mmol) was dissolved in dichloromethane (10 mL), and heptadecan-9-yl 3-hydroxypropanoate 15b (658 mg, 2.0 mmol), DMAP (84 mg, 0.8 mmol), and triethylamine (248 mg, 2.4 mmol) were added. The mixture was stirred for dissolution. A solution of EDC.HCL (440 mg, 2.4 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15c (528 mg, light yellow oil, yield: 57%).

MS m/z (ESI): 463.2 [M+1]

### Step 4: Synthesis of compound 15d

At room temperature, compound 15c (528 mg, 1.14 mmol) was dissolved in ethanol (10 mL), and butanolamine (1.5 g, 17 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 15d (480 mg, light yellow oil).

MS m/z (ESI): 472.4 [M+1]

### Step 5: Synthesis of compound 15e

3-Oxopropanoic acid (0.44 g, 5.0 mmol) was dissolved in dichloromethane (20 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15a (1.03 g, light yellow oil, yield: 45%).

MS m/z (ESI): 456.4 [M+1]

### Step 6: Synthesis of compound 15f

Compound 15e (1.03 g, 2.2 mmol) was dissolved in n-butanol (10 mL), and sodium borohydride (266 mg, 7 mmol) was added at 0 °C. The mixture was stirred at room temperature for 12 h. After the starting materials were consumed completely as monitored, a saturated ammonium chloride solution was added to quench the reaction, and the resulting mixture was extracted with dichloromethane. The organic phase was dried over anhydrous sodium sulfate and concentrated to give compound 15f (980 mg, light yellow oil).

MS m/z (ESI): 459.4 [M+1]

### Step 7: Synthesis of compound 15g

3-Bromopropanoic acid (306 mg, 2.0 mmol) was dissolved in dichloromethane (10 mL), and compound 15f (916 mg, 2.0 mmol), DMAP (84 mg, 0.8 mmol), and triethylamine (248 mg, 2.4 mmol) were added. The mixture was stirred for dissolution. A solution of EDC.HCL (440 mg, 2.4 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15g (629 mg, light yellow oil, yield: 53%).

MS m/z (ESI): 593.3 [M+1]

### Step 8: Synthesis of compound 15

Compound 15d (480 mg, 1.0 mmol) was dissolved in tetrahydrofuran, and acetonitrile, compound 15g (600 mg, 1.1 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 15 (384 mg, light yellow oil, yield: 39%).
MS m/z (ESI): 984.8 [M+1]
1H NMR (300MHz, CDCl3): δ 5.40 (t, 1H, J = 5.4 Hz), 4.53-4.30 (m, 6H), 3.77-3.71 (m, 4H), 3.47-3.31 (m, 2H), 3.12-3.05 (m, 2H), 2.68-2.60 (m, 8H), 2.37-2.25 (m, 6H), 2.10-0.85 (m, 72H), 0.70 (s, 3H)

### Example 16. Synthesis of Compound 16

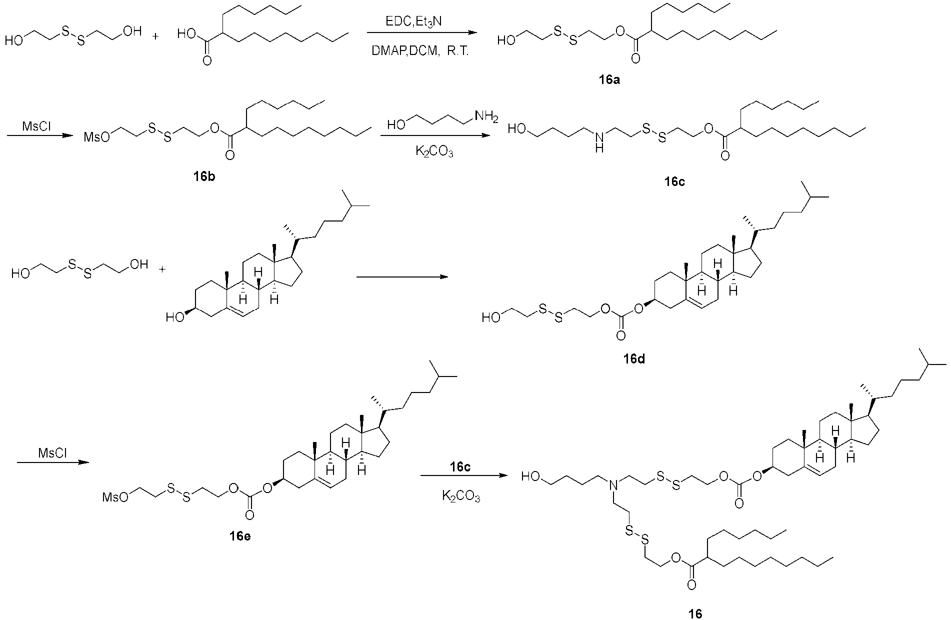

### Step 1: Synthesis of compound 16a

2-Hexyldecanoic acid (1.28 g, 5.0 mmol) was dissolved in dichloromethane (20 mL), and bis(2-hydroxyethyl)disulfide (1.54 g, 10.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated, and the residue was subjected to column chromatography to give compound 16a (1.00 g, light yellow oil, yield: 51%).

MS m/z (ESI): 393.2 [M+1]

### Step 2: Synthesis of compound 16b

Compound 16a (1.00 g, 2.5 mmol) was dissolved in dichloromethane (20 mL), and triethylamine (0.76 g, 7.5 mmol) was added. The reaction mixture was cooled to 0 °C in an ice-water bath, followed by addition of methanesulfonyl chloride (0.72 g, 6.2 mmol). The resulting mixture was stirred at room temperature for 16-20 h. After the reaction was completed, the mixture was washed twice with saturated sodium bicarbonate and washed twice with water. The organic phase was dried over anhydrous sodium sulfate, filtered, and concentrated to give compound 16b (1.00 g, light yellow oil, yield: 85%).

MS m/z (ESI): 471.2 [M+1]

### Step 3: Synthesis of compound 16c

Compound 16b (1.00 g, 2.0 mmol) was dissolved in acetonitrile (20 mL), and potassium carbonate (0.83 g, 6.0 mmol) and 4-aminobutanol (0.18 g, 2.0 mmol) were added. The mixture was stirred at room temperature for 16-20 h. After the reaction was completed, the mixture was filtered through celite. Dichloromethane was added to the filtrate, and the resulting mixture was washed twice with saturated sodium bicarbonate and washed twice with water. The organic phase was dried over anhydrous sodium sulfate, filtered, and concentrated to give compound 16c (658 mg, light yellow oil, yield: 71%).

MS m/z (ESI): 464.3 [M+1]

### Step 4: Synthesis of compound 16d

Cholesterol (1.93 g, 5.0 mmol) was dissolved in dichloromethane (20 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Bis(2-hydroxyethyl) disulfide (0.86 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 16d (1.45 g, light yellow oil, yield: 51%).

MS m/z (ESI): 567.4 [M+1]

### Step 5: Synthesis of compound 16e

Compound 16d (1.42 g, 2.5 mmol) was dissolved in dichloromethane (20 mL), and triethylamine (0.76 g, 7.5 mmol) was added. The reaction mixture was cooled to 0 °C in an ice-water bath, followed by addition of methanesulfonyl chloride (0.72 g, 6.2 mmol). The resulting mixture was stirred at room temperature for 16-20 h. After the reaction was completed, the mixture was washed twice with saturated sodium bicarbonate and washed twice with water. The organic phase was dried over anhydrous sodium sulfate, filtered, and concentrated to give compound 16e (1.26 g, light yellow oil, yield: 78%).

MS m/z (ESI): 645.3 [M+1]

### Step 6: Synthesis of compound 16

Compound 16e (1.26 g, 2.0 mmol) was dissolved in acetonitrile (20 mL), and potassium carbonate (0.83 g, 6.0 mmol) and compound 16c (0.93 g, 2.0 mmol) were added. The mixture was stirred at room temperature for 16-20 h. After the reaction was completed, the mixture was filtered through celite. Dichloromethane was added to the filtrate, and the resulting mixture was washed twice with saturated sodium bicarbonate and washed twice with water. The organic phase was dried over anhydrous sodium sulfate, filtered, and concentrated, and the residue was subjected to column chromatography to give compound 16 (871 mg, light yellow oil, yield: 43%).
MS m/z (ESI): 1012.7 [M+1]
1H NMR (300MHz, CDCl3): δ 5.40 (t, 1H, J = 5.4 Hz), 4.53-4.40 (m, 1H), 4.20-4.02 (m, 4H), 3.67-3.61 (m, 2H), 3.58-3.50 (m, 2H), 2.47-2.42 (m, 8H), 2.03-1.72 (m, 5H), 1.67-0.85 (m, 74H), 0.70 (s, 3H)

### Example 17. Synthesis of Compound 17

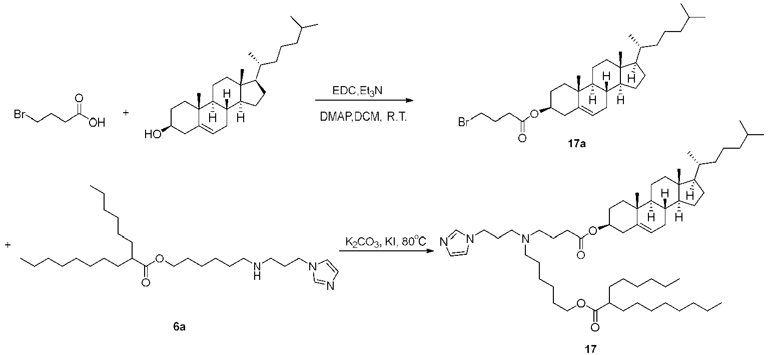

### Step 1: Synthesis of compound 17a

4-Bromobutyric acid (0.84 g, 5.0 mmol) was dissolved in dichloromethane (30 mL), and cholesterol (1.94 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 17a (1.68 g, light yellow oil, yield: 63%).

MS m/z (ESI): 535.3 [M+1]

### Step 2: Synthesis of compound 17

Compound 6a (463 mg, 1.0 mmol) was dissolved in tetrahydrofuran, and acetonitrile, compound 17a (643 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 17 (505 mg, light yellow oil, yield: 55%).
MS m/z (ESI): 918.9 [M+1]
1H NMR (300MHz, CDCl3): δ 7.49 (s, 1H), 7.08 (s, 1H), 6.94 (s, 1H), 5.40 (t, 1H, J = 5.4 Hz), 4.53-4.40 (m, 1H), 4.10-3.92 (m, 4H), 2.48-2.25 (m, 11H), 2.10-0.85 (m, 80H), 0.70 (s, 3H)

### Example 18. Synthesis of Compound 18

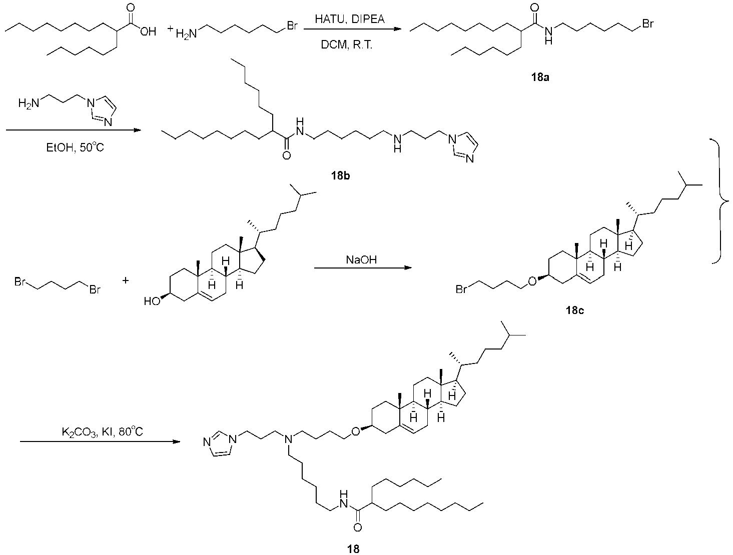

### Step 1: Synthesis of compound 18a

2-Hexyldecanoic acid (2.12 g, 5.0 mmol) was dissolved in dichloromethane (30 mL), and 6-bromohexylamine hydrochloride (1.09 g, 5.0 mmol), HATU (2.28 g, 6.0 mmol), and DIPEA (1.29 g, 10.0 mmol) were added. The mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 18a (1.40 g, light yellow oil, yield: 67%).

MS m/z (ESI): 418.3 [M+1]

### Step 2: Synthesis of compound 18b

At room temperature, compound 18a (1.25 g, 3 mmol) was dissolved in ethanol (20 mL), and 1-(3-aminopropyl)imidazole (5.63 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 18b (1.42 g, light yellow oil).

MS m/z (ESI): 463.4 [M+1]

### Step 3: Synthesis of compound 18c

1,4-Dibromobutane (2.16 g, 10.0 mmol) was dissolved in n-butanol (30 mL), and cholesterol (1.94 g, 5.0 mmol) and sodium hydroxide (1.20 g, 30 mmol) were added. The mixture was heated to reflux and stirred for 5 h. After the reaction was completed, diluted hydrochloric acid was added to adjust the pH to neutral. n-Butanol was removed by rotary evaporation, and dichloromethane (20 mL) was added for dilution. The resulting mixture was then washed with saturated brine (30 mL). The organic phase was dried over anhydrous sodium sulfate, filtered, and concentrated, and the residue was subjected to column chromatography to give product 18c (1.25 g, light yellow oil, yield: 48%).

MS m/z (ESI): 521.3 [M+1]

### Step 4: Synthesis of compound 18

Compound 18b (463 mg, 1.0 mmol) was dissolved in tetrahydrofuran, and acetonitrile, compound 18c (625 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product 18 (434 mg, light yellow oil, yield: 48%).
MS m/z (ESI): 903.9 [M+1]
1H NMR (300MHz, CDCl₃): δ 8.01 (s, 1H), 7.49 (s, 1H), 7.08 (s, 1H), 6.94 (s, 1H), 5.40 (t, 1H, J = 5.4 Hz), 4.10-3.92 (m, 2H), 3.35 (t, 2H, J = 5.4 Hz), 3.28-3.01 (m, 7H), 2.50-2.42 (m, 4H), 2.10-0.85 (m, 83H), 0.70 (s, 3H)

### Example 19. Synthesis of Compound 19

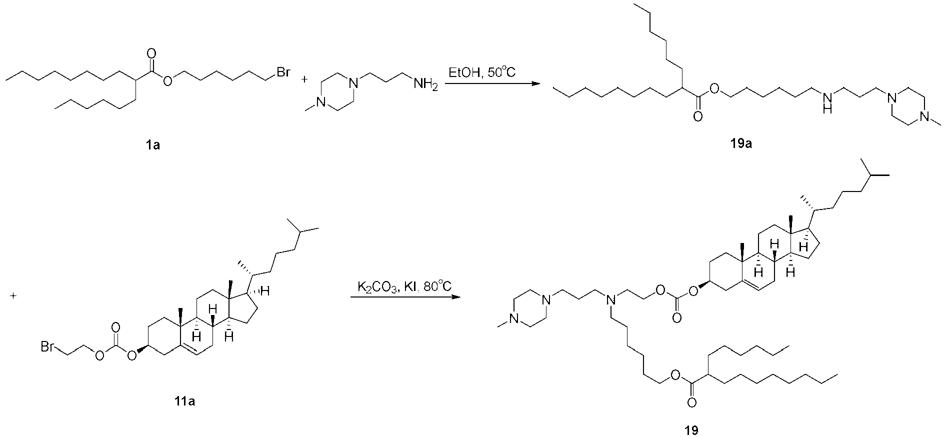

### Step 1: Synthesis of compound 19a

At room temperature, 6-bromohexyl 2-hexyldecanoate 1a (1.28 g, 3 mmol) was dissolved in ethanol (20 mL), and 1-(3-aminopropyl)-4-methylpiperazine (7.07 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 19a (1.49 g, light yellow oil).

MS m/z (ESI): 496.5 [M+1]

### Step 2: Synthesis of compound 19

Compound 19a (496 mg, 1.0 mmol) was dissolved in tetrahydrofuran, and acetonitrile, compound 11a (646 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 19 (543 mg, light yellow oil, yield: 57%).
MS m/z (ESI): 952.9 [M+1]
1H NMR (300MHz, CDCl₃): δ 5.40 (t, 1H, J = 5.4 Hz), 4.53-4.40 (m, 1H), 4.20-3.95 (m, 6H), 2.74-2.65 (m, 2H), 2.47-2.40 (m, 6H), 2.36-0.85 (m, 90H), 0.70 (s, 3H)

### Example 20. Synthesis of Compound 20

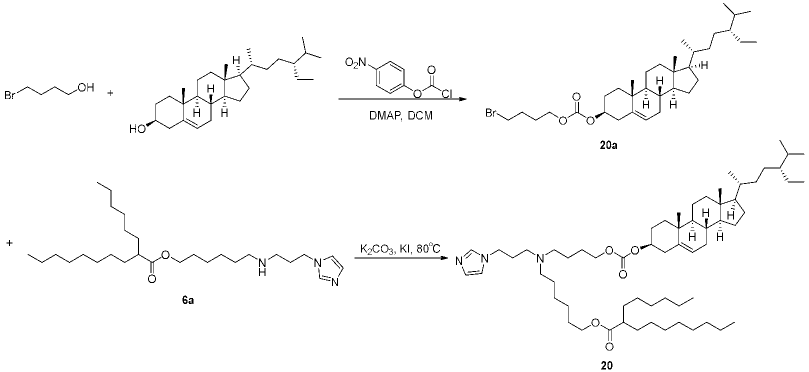

### Step 1: Synthesis of compound 20a

4-Bromobutanol (0.77 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. β-Sitosterol (2.32 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 20a (1.63 g, light yellow oil, yield: 55%).

MS m/z (ESI): 593.3 [M+1]

### Step 2: Synthesis of compound 20

6-((3-(1H-Imidazol-1-yl)propyl)amino)hexyl 2-hexyldecanoate 6a (463 mg, 1.0 mmol) was dissolved in tetrahydrofuran, and acetonitrile, compound 20a (713 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 20 (547 mg, light yellow oil, yield: 56%).
MS m/z (ESI): 976.9 [M+1]
1H NMR (300MHz, CDCl₃): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 6H), 2.47-2.30 (m, 8H), 2.36-0.85 (m, 87H), 0.70 (s, 3H)

### Example 21. Synthesis of Compound 21

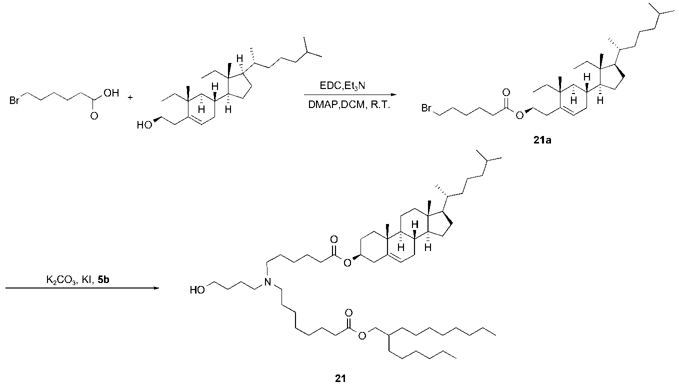

### Step 1: Synthesis of compound 21a

6-Bromohexanoic acid (0.975 g, 5.0 mmol) was dissolved in dichloromethane (30 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 21a (1.92 g, light yellow oil, yield: 68%).

MS m/z (ESI): 563.4 [M+1]

### Step 2: Synthesis of compound 21

2-Hexyldecyl 8-((4-hydroxybutyl)amino)octanoate 5b (455 mg, 1.0 mmol) was dissolved in tetrahydrofuran, and acetonitrile, cholesterol 6-bromohexanoate 21a (676 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 21 (422 mg, light yellow oil, yield: 45%).
MS m/z (ESI): 938.9 [M+1]
1H NMR (400 MHz, CDCl₃ ) δ 5.27 (tt, J = 12.5, 2.0 Hz, 1H), 4.64 (p, J = 14.8 Hz, 1H), 4.31 (ddd, J = 103.0, 24.8, 14.1 Hz, 2H), 3.46 (t, J = 14.8 Hz, 2H), 2.78 - 2.63 (m, 6H), 2.44 - 2.27 (m, 4H), 2.27 - 1.47 (m, 21H), 1.45 - 1.15 (m, 47H), 1.15 - 0.96 (m, 4H), 0.95 - 0.83 (m, 21H).

### Example 22. Synthesis of Compound 24

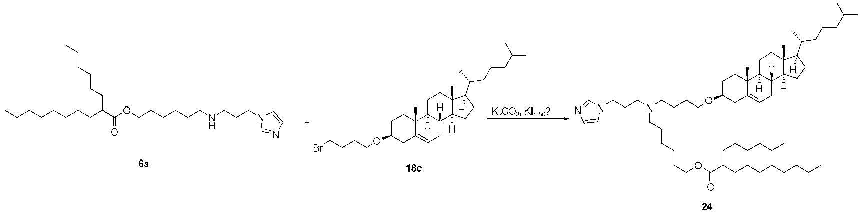

Compound 6a (463 mg, 1.0 mmol) was dissolved in tetrahydrofuran, and acetonitrile, compound 18c (625 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product **24** (470 mg, light yellow oil, yield: 52%).
MS m/z (ESI): 904.9 [M+1]
1H NMR (400 MHz, CDCl₃ ) δ 7.92 (s, 1H), 7.24 - 6.71 (m, 2H), 5.27 (tt, J = 12.5, 2.0 Hz, 1H), 4.17 - 3.98 (m, 4H), 3.57 - 3.31 (m, 3H), 2.79 - 2.63 (m, 4H), 2.47 - 2.38 (m, 4H), 2.37 - 1.77 (m, 7H), 1.75 - 1.00 (m, 58H), 0.96 - 0.81 (m, 21H).

### Example 23. Synthesis of Compound 25

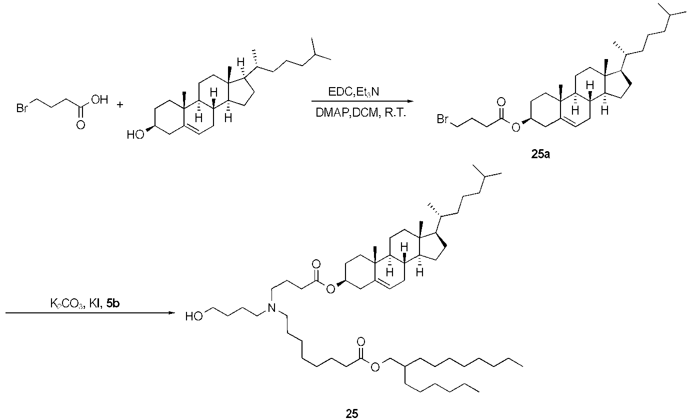

### Step 1: Synthesis of compound 25a

4-Bromobutyric acid (0.835 g, 5.0 mmol) was dissolved in dichloromethane (30 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 25a (1.90 g, light yellow oil, yield: 71%).

MS m/z (ESI): 535.4 [M+1]

### Step 2: Synthesis of compound 25

2-Hexyldecyl 8-((4-hydroxybutyl)amino)octanoate 5b (455 mg, 1.0 mmol) was dissolved in tetrahydrofuran, and acetonitrile, cholesterol 4-bromobutyrate 21a (643 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 25 (373 mg, light yellow oil, yield: 41%).
MS m/z (ESI): 910.9 [M+1]
1H NMR (400 MHz, CDCl₃) δ 5.27 (tt, J = 12.5, 2.0 Hz, 1H), 4.68 (p, J = 14.8 Hz, 1H), 4.31 (ddd, J = 103.0, 24.8, 13.0 Hz, 2H), 3.46 (t, J = 14.8 Hz, 2H), 2.79 - 2.62 (m, 4H), 2.43 (td, J = 12.2, 6.0 Hz, 4H), 2.37 - 2.11 (m, 3H), 2.11 - 1.80 (m, 9H), 1.79 - 1.46 (m, 11H), 1.45 - 1.15 (m, 43H), 1.14 - 0.98 (m, 4H), 0.97 - 0.80 (m, 21H).

### Example 24: mRNA- Encapsulation by Three-Component LNP Compositions and Characterization of LNP-mRNA

### (1) Encapsulation of mRNA by LNP compositions

Taking GFP mRNA (green fluorescent protein) as an example, the construction of steroid-cationic lipid three-component LNP-mRNA vaccines and four-component LNP-mRNA vaccines (the three-component vaccines did not contain cholesterol; the four-component vaccines contained additional cholesterol) was displayed. The steroid-cationic lipid compound of the present disclosure, a helper lipid (neutral phospholipid), a polyethylene glycol lipid, and cholesterol (if present) were dissolved in an ethanol solution according to the molar ratio shown in Table 1 to obtain a lipid mixture; the mRNA (GFP mRNA, green fluorescent protein) was dissolved in an acetic acid buffer at pH 4.0; and the lipid mixture and the mRNA solution were loaded onto a microfluidic device nanoparticle preparation instrument to prepare an mRNA-LNP composition (the flow rate ratio of the lipid mixture to the mRNA was 1:3).

### (2) Characterization of LNP-mRNA

The packaged mRNA-LNPs were dialyzed and concentrated by ultrafiltration into DPBS, and the resulting mixtures were sterilely filtered to obtain samples for subsequent animal experiments. The samples were taken and analyzed for encapsulation efficiency, average particle size, PDI, and Zeta potential.

**Table 1. Molar percentages and nitrogen-to-phosphorus ratios of lipid formulations of three-component LNPs formed from steroid-cationic lipid compounds**

| No. | Lipid formulation (molar percentage) | Nitrogen-to-phosp horus ratio |
|---|---|---|
| LNP1 | Compound 1:DOPE:DMG-PEG2000 = 49.25:49.25:1.5 | 6 |
| LNP2 | Compound 2:DOPE:DMG-PEG2000 = 49.25:49.25:1.5 | 4 |
| LNP3 | Compound 3:DOPE:DMG-PEG2000 = 49.25:49.25:1.5 | 8 |
| LNP4 | Compound 4:DOPE:DMG-PEG2000 = 49.25:49.25:1.5 | 6 |
| LNP5 | Compound 5:DOPE:DMG-PEG2000 = 49.25:49.25:1.5 | 6 |
| LNP6 | Compound 6:DOPE:DMG-PEG2000 = 49.25:49.25:1.5 | 8 |
| LNP7 | Compound 7:DOPE:DMG-PEG2000 = 49.25:49.25:1.5 | 6 |
| LNP8 | Compound 8:DOPE:DMG-PEG2000 = 49.25:49.25:1.5 | 6 |
| LNP9 | Compound 9:DOPE:DMG-PEG2000 = 49.25:49.25:1.5 | 8 |
| LNP10 | Compound 10:DOPE:DMG-PEG2000 = 49.25:49.25:1.5 | 8 |
| LNP11 | Compound 11:DOPE:DMG-PEG2000 = 49.25:49.25:1.5 | 8 |
| LNP12 | Compound 12:DOPE:DMG-PEG2000 = 49.25:49.25:1.5 | 6 |
| LNP13 | Compound 13:DOPE:DMG-PEG2000 = 49.25:49.25:1.5 | 6 |
| LNP14 | Compound 14:DOPE:DMG-PEG2000 = 49.25:49.25:1.5 | 6 |
| LNP15 | Compound 15:DOPE:DMG-PEG2000 = 49.25:49.25:1.5 | 6 |
| LNP16 | Compound 16:DOPE:DMG-PEG2000 = 49.25:49.25:1.5 | 6 |
| LNP17 | Compound 17:DOPE:DMG-PEG2000 = 49.25:49.25:1.5 | 6 |
| LNP18 | Compound 18:DOPE:DMG-PEG2000 = 49.25:49.25:1.5 | 6 |
| LNP19 | Compound 19:DOPE:DMG-PEG2000 = 49.25:49.25:1.5 | 10 |
| LNP20 | Compound 20:DOPE:DMG-PEG2000 = 49.25:49.25:1.5 | 6 |
| LNP21 | Compound 1:DSPC:DMG-PEG2000 = 73.87:24.62:1.5 | 6 |
| LNP22 | Compound 1:DSPC:DMG-PEG2000 = 65.7:32.8:1.5 | 6 |
| LNP23 | Compound 2:DOPE:DMG-PEG2000 = 73.87:24.62:1.5 | 4 |
| LNP24 | Compound 6:DSPC:DMG-PEG2000 = 49.25:49.25:1.5 | 6 |
| LNP25 | Compound 6:DOPE:DMG-PEG2000 = 65:33:2 | 6 |
| LNP26 | Compound 10:DOPE:DMG-PEG2000 = 47.25:47.25:5 | 8 |
| LNP27 | Compound 11:DSPC:DMG-PEG2000 = 49.25:49.25:1.5 | 8 |
| LNP28 | Compound 11:DOPE:DMG-PEG2000 = 65:33:2 | 8 |
| LNP A | Compound (1) of patent CNI12424214A:DOPE:DMG-PEG2000 = 49.25:49.25:1.5 | 6 |
| Control group A | Compound (1) of patent CN112424214A:cholesterol:DOPE:DMG-PEG2000 = 20:25:50:5 | 6 |
| LNP B | Compound (2) of patent CN112424214A:DOPE:DMG-PEG2000 = 49.25:49.25:1.5 | 6 |
| Control group B | Compound (2) of patent CN112424214A:cholesterol:DOPE:DMG-PEG2000 = 20:25:50:5 | 6 |
| LNP C | Compound (3) of patent CN112424214A:DOPE:DMG-PEG2000 = 49.25:49.25:1.5 | 6 |
| Control group C | Compound (3) of patent CN112424214A:cholesterol:DOPE:DMG-PEG2000 = 20:25:50:5 | 6 |
| LNP D | Compound of patent US7514099B2 (CLinDMA):DOPE:DMG-PEG2000 = 49.25:49.25:1.5 | 6 |
| Control group D | Compound of patent US7514099B2 (CLinDMA):cholesterol:DOPE:DMG-PEG2000 = 20:25:50:5 | 6 |
| Control group E | ALC-0315:DSPC:cholesterol:DMG-PEG2000 = 50:10:38.5:1.5 | 6 |

Among them, compound (1) of patent CN112424214A:

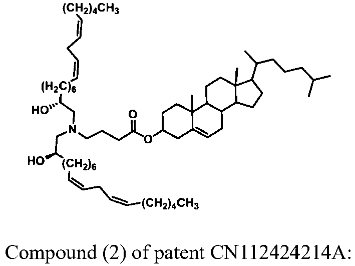

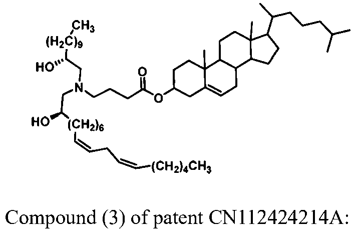

## Claims

1. A lipid nanoparticle composition for nucleic acid drug delivery, comprising: a steroid-cationic lipid compound, a helper lipid, and a polyethylene glycol lipid.

2. The lipid nanoparticle composition for nucleic acid drug delivery according to claim 1, wherein the molar ratio of the steroid-cationic lipid to the helper lipid to the polyethylene glycol lipid is (30-80):(20-80):(0.5-20).

3. The lipid nanoparticle composition for nucleic acid drug delivery according to claim 1, wherein the molar ratio of the steroid-cationic lipid to the helper lipid to the polyethylene glycol lipid is (30-80):(30-80):(0.5-20).

4. The lipid nanoparticle composition for nucleic acid drug delivery according to claim 1, wherein the polyethylene glycol lipid is selected from 2-[(polyethylene glycol)-2000]-N,N-ditetradecylacetamide (ALC-0159), 1,2-dimyristoyl-sn-glycero-methoxypolyethylene glycol (PEG-DMG), 1,2-distearoyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearoyl, PEG-diacylglycerol amide (PEG-DAG), PEG-dipalmitoyl phosphatidylethanolamine (PEG-DPPE), and PEG-1,2-dimyristoyloxypropyl-3-amine (PEG-c-DMA).

5. The lipid nanoparticle composition for nucleic acid drug delivery according to claim 1, wherein the helper lipid is selected from a neutral lipid, a zwitterionic lipid, and an anionic lipid; preferably, the helper lipid is selected from 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoylphosphatidylcholine (POPC), 1-palmitoyl-2-oleoylphosphatidylethanolamine (POPE), phosphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauroylphosphatidylcholine (DLPC), dimyristoylphosphatidylcholine (DMPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1,2-diarachidoyl-sn-glycero-3-phosphocholine (DBPC), 1-stearoyl-2-palmitoylphosphatidylcholine (SPPC), 1,2-eicosenoyl-sn-glycero-3-phosphocholine (DEPC), lysophosphatidylcholine, dilinoleoylphosphatidylcholine distearoylphosphatidylethanolamine (DSPE), and lysophosphatidylethanolamine.

6. The lipid nanoparticle composition for nucleic acid drug delivery according to claim 1, wherein the composition further comprises a nucleic acid drug; the nucleic acid drug is a DNA or an RNA; the RNA is selected from an antisense RNA, an saRNA, an mRNA, an lncRNA, an miRNA, an siRNA, a piRNA, a gRNA, a tsRNA, a circRNA, and a self-amplifying mRNA; preferably, the nucleic acid drug is an mRNA; more preferably, the composition is in the form of a nucleic acid drug-lipid nanoparticle.

7. The lipid nanoparticle composition for nucleic acid drug delivery according to any one of claims 1-6, wherein the steroid-cationic lipid compound has a structure represented by formula (I): 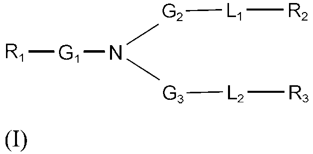
wherein R₁ is selected from -OR₄, -NR₄R₅, -NR₄C(=O)R₅, -C(=O)OR₅, -OC(=O)R₅, - OC(=O)OR₅, -CN, a nitrogen-containing heterocyclic group, and guanidino;
R₄ and R₅ are each independently selected from H, C₁₋₉ alkyl, C₂₋₉ alkenyl, C₂₋₉ alkynyl, C₃₋₈ cycloalkyl, C₃₋₈ cycloalkenyl, and C₃₋₈ cycloalkynyl;
G₁ is selected from a chemical bond (-), C₁₋₉ alkylene, C₂₋₉ alkenylene, C₃₋₉ alkynylene, C₃₋₈ cycloalkylene, and C₃₋₈ cycloalkenylene;
preferably, R₁-G₁- has no more than 10 contiguous carbon atoms;
G₂ and G₃ are each independently selected from a chemical bond (-), C₁₋₁₂ alkylene, C₂₋₁₂ alkenylene, C₂₋₁₂ alkynylene, C₃₋₁₂ cycloalkylene, C₃₋₁₂ cycloalkenylene, C₃₋₁₂ cycloalkynylene, and C₆₋₁₂ arylene;
L₁ and L₂ are each independently selected from one or a combination of two or more of a chemical bond (-), -O-, -S-, -O(C=O)O-, -(C=O)NRₐ-, -NRₐ(C=O)-, -O(C=O)-, -(C=O)O-, -S-S-, -S(O)ₓ-, -OS(O)ₓO-, -C(=O)S-, -SC(=O)-, -NRₐC(=O)NRb-, -OC(=O)NRₐ-, -NRₐC(=O)O-, -OC(=O)S-, - SC(=O)O-, -P(O)(ORₐ)O-, -OP(O)(ORₐ)O-, and C₁₋₁₂ alkylene;
wherein x is selected from 0, 1, and 2;
wherein Rₐ and R_{b} are each independently selected from H, C₁₋₁₂ alkyl, C₂₋₁₂ alkenyl, and C₂₋₁₂ alkyne;
R₂ is selected from a naturally occurring or non-naturally occurring steroid;
R₃ is selected from a naturally occurring or non-naturally occurring steroid, C₆₋₂₄ alkyl, C₆₋₂₄ alkenyl, C₆₋₂₄ alkyne, and C₆₋₂₄ alkoxy.

8. The lipid nanoparticle composition for nucleic acid drug delivery according to claim 7, wherein R₁ is selected from -OR₄, -NR₄R₅, pyrazolyl, imidazolyl, piperazinyl, alkylpiperazine, piperidinyl, alkylpiperidine, guanidino, pyrrolyl, and pyrrolidinyl; R₄ and R₅ are each independently selected from H, C₁₋₉ alkyl, C₂₋₉ alkenyl, C₂₋₉ alkynyl, C₃₋₈ cycloalkyl, and C₃₋₈ cycloalkenyl;
G₁ is a chemical bond (-), C₁₋₉ alkylene, C₂₋₉ alkenylene, C₃₋₈ cycloalkylene, or C₃₋₈ cycloalkenylene;
preferably, G₁ is C₁₋₆ alkylene or C₂₋₆ alkenylene;
G₂ and G₃ are each independently selected from a chemical bond (-), C₁₋₁₂ alkylene, C₂₋₁₂ alkenylene, and C₂₋₁₂ alkynylene;
L₁ and L₂ are each independently selected from one or a combination of two or more of -O(C=O)-, -(C=O)O-, -S-S-, -O(C=O)O-, -NRₐC(=O)O-, -(C=O)NRₐ-, -NRₐ(C=O)-, -C(=O)S-, -SC(=O)-, - OC(=O)NRₐ-, and C₁₋₁₂ alkylene;
wherein Rₐ is H, C₁₋₁₂ alkyl, C₂₋₁₂ alkenyl, or C₂₋₁₂ alkyne.

9. The lipid nanoparticle composition for nucleic acid drug delivery according to claim 7, wherein R₁ is selected from -OR₄, -NR₄R₅, imidazolyl, piperazinyl, and guanidino;
R₄ and R₅ are each independently selected from H and C₁₋₅ alkyl;
G₁ is unsubstituted C₁₋₆ alkylene;
G₂ and G₃ are each independently selected from a chemical bond (-) and C₁₋₁₀ alkylene;
L₁ and L₂ are each independently selected from -O-, -O(C=O)-, -O(C=O)O-, -(C=O)O-, -(C=O)S-, -NHC(=O)O-, -NHC(=O)-, 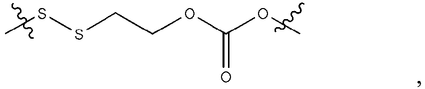 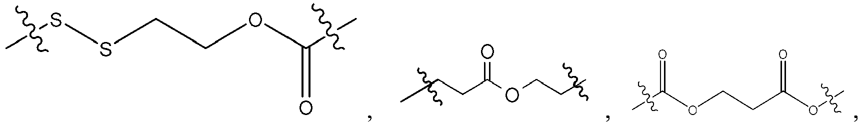 and

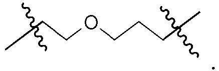

10. The lipid nanoparticle composition for nucleic acid drug delivery according to claim 7, wherein R₂ is a sterol;
preferably, the sterol is a zoosterol or an oxidized or reduced form thereof; and/or the sterol is a phytosterol or an oxidized or reduced form thereof; and/or the sterol is a synthetic sterol or an oxidized or reduced form thereof;
more preferably, the sterol is selected from cholesterol, an oxidized form of cholesterol, a reduced form of cholesterol, alkyl lithocholate, stigmasterol, stigmastanol, campesterol, ergosterol, and sitosterol;
more preferably, the sterol is an oxidized form of cholesterol, a reduced form of cholesterol, alkyl lithocholate, stigmasterol, stigmastanol, campesterol, ergosterol, or sitosterol;
more preferably, the sterol is selected from avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, and lithocholic acid;
more preferably, the sterol has the following structural formula: 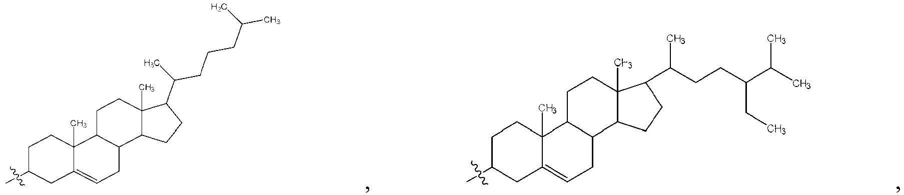 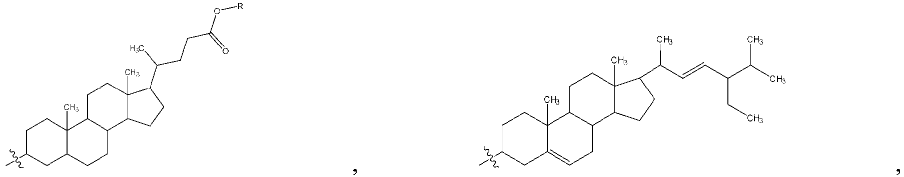 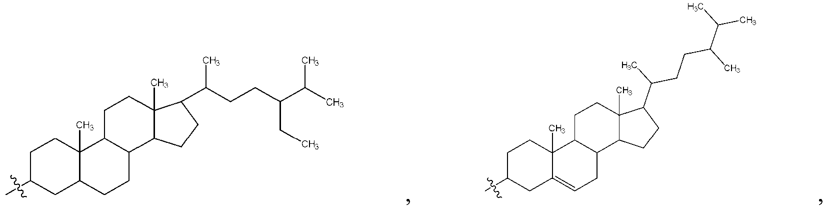 or 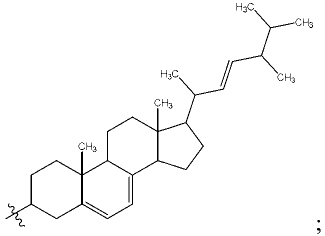
wherein R is C₁₋₂₀ alkyl.

11. The lipid nanoparticle composition for nucleic acid drug delivery according to claim 7, wherein
R₃ is selected from: 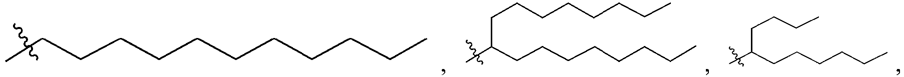 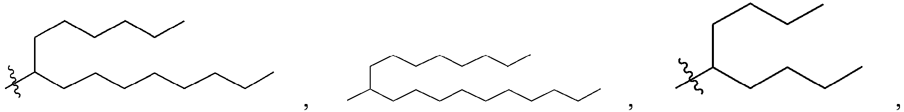 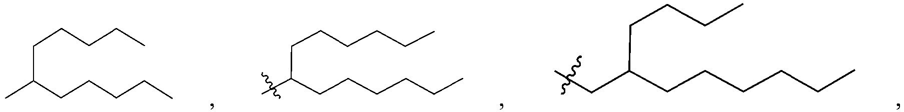 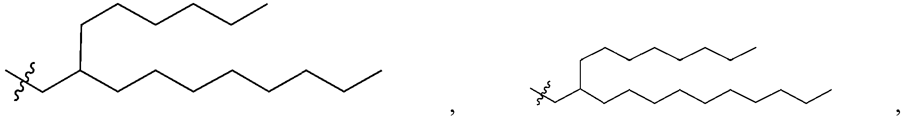 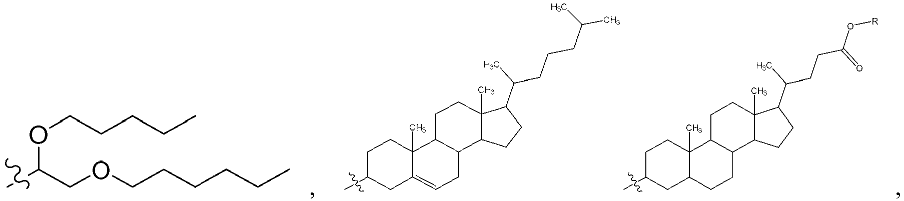 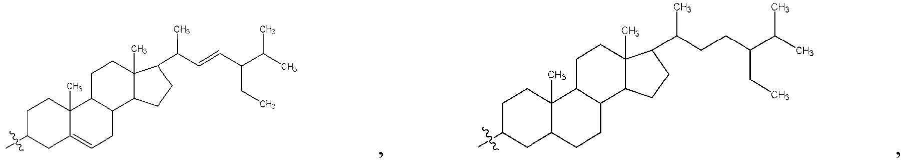 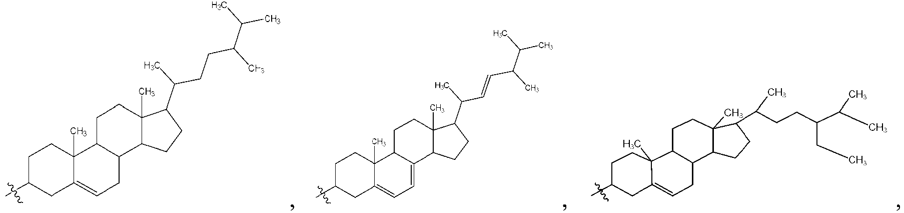 and 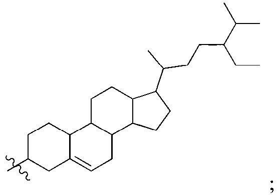
preferably, R₃ is selected from: 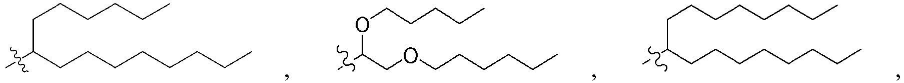 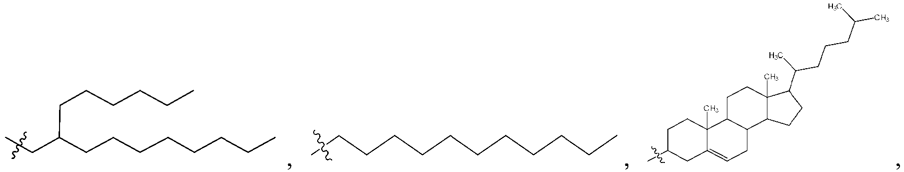 and

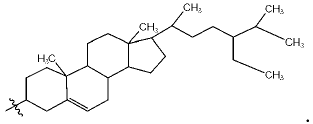

12. The lipid nanoparticle composition for nucleic acid drug delivery according to claim 7,
wherein
the compound is selected from: 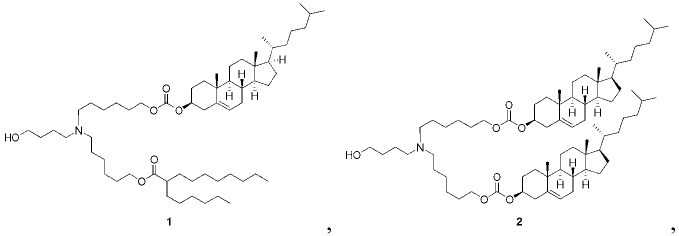 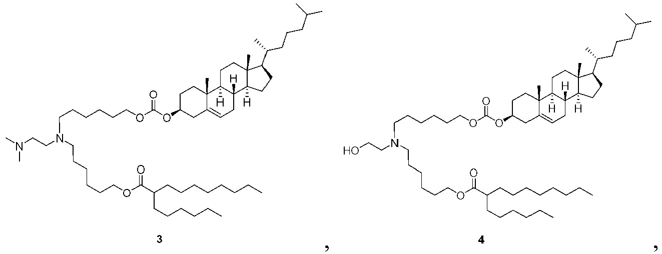 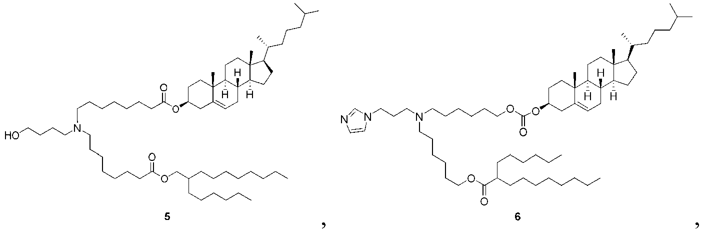 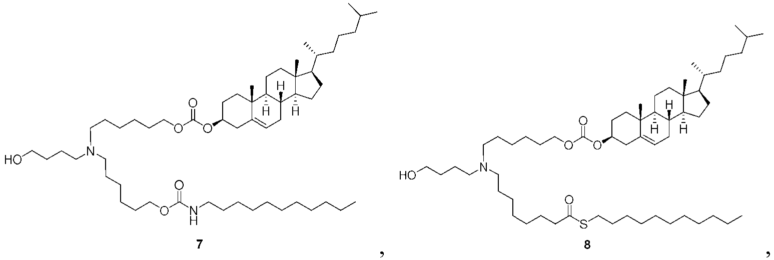 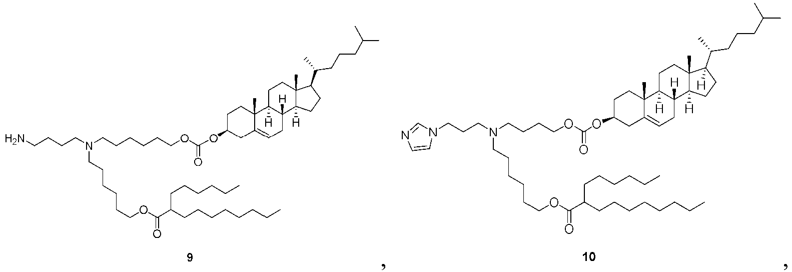 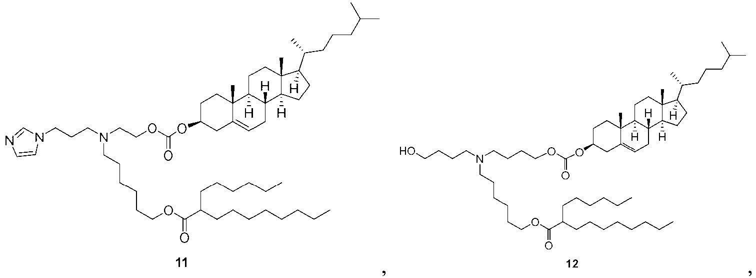 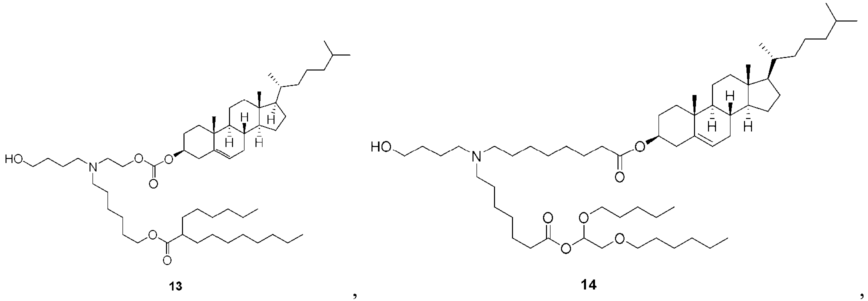 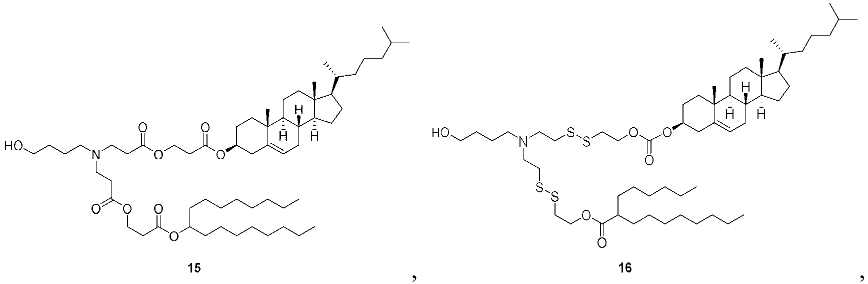 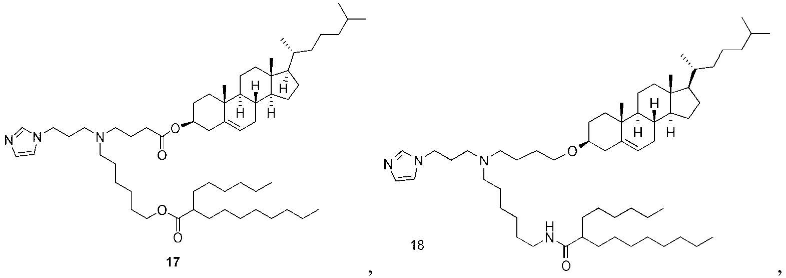 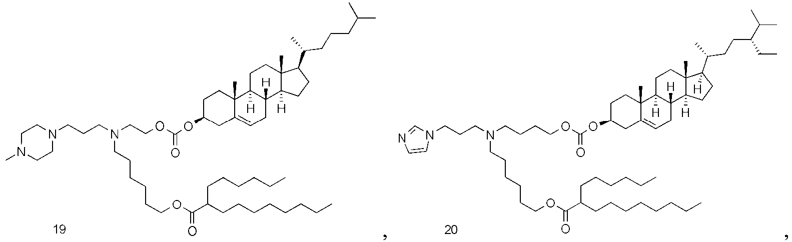 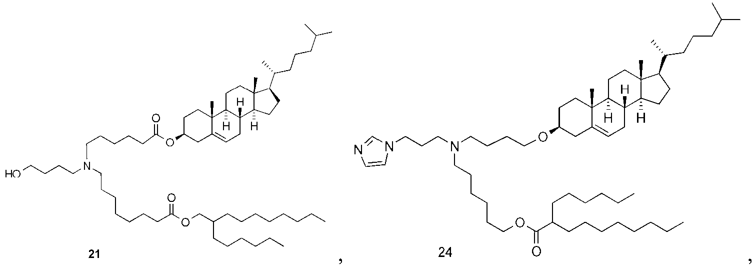 and

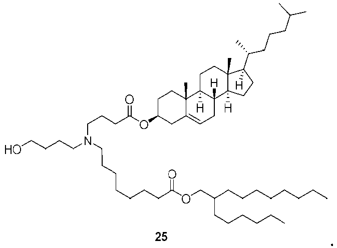

13. The lipid nanoparticle composition for nucleic acid drug delivery according to any one of claims 6-12, wherein a nitrogen-to-phosphorus ratio of the lipid nanoparticle is (1-15):1; preferably, the nitrogen-to-phosphorus ratio of the lipid nanoparticle is (1-10):1.

14. The lipid nanoparticle composition for nucleic acid drug delivery according to any one of claims 1-12, wherein the lipid nanoparticle has a diameter of 15-300 nm.

15. The lipid nanoparticle composition for nucleic acid drug delivery according to any one of claims 1-12, wherein a dosage form of the composition is a liquid formulation or a lyophilized powder formulation.

16. A preparation method for the lipid nanoparticle composition for nucleic acid drug delivery according to any one of claims 6-12, comprising: dissolving a steroid-cationic lipid compound, a helper lipid, and a polyethylene glycol lipid in a solvent and then mixing with a nucleic acid drug to give the lipid nanoparticle composition for nucleic acid drug delivery.

17. Use of the lipid nanoparticle composition according to any one of claims 1-12 in preparing a bioactive substance delivery system.

18. The use according to claim 17, wherein the bioactive substance delivery system is an aerosol inhalation delivery system.

19. The use according to claim 18, wherein the aerosol inhalation delivery system is administered by nebulizing a liquid formulation, and/or the aerosol inhalation delivery system is administered by transmucosal administration; preferably, the aerosol inhalation delivery system is administered by nasal inhalation or oral inhalation.

20. The use according to claim 18 or 19, wherein the delivery system further comprises a pharmaceutically acceptable excipient; preferably, the excipient comprises one or a combination of two or more of sodium acetate, tromethamine, potassium dihydrogen phosphate, sodium chloride, disodium hydrogen phosphate, and sucrose.

21. The use according to claim 20, wherein the delivery system is a vaccine.

22. The use according to claim 21, wherein the vaccine is a vaccine for preventing a cancer, a virus infection, a bacterial infection, or a fungal infection; preferably, the virus is selected from: norovirus, Ebola virus, coronavirus, cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, and measles virus.

23. Use of the lipid nanoparticle composition according to any one of claims 1-12 in preparing a lyophilized formulation.
